# EUROPEAN PATENT APPLICATION

(11) **EP 2 907 464 A1**
(43) Date of publication of application: **19.08.2015**
(21) Application number: 15154865.8
(22) Date of filing: 12.02.2015
(51) Int. Cl.: A61B 18/14, A61N 7/02

(54) **Methods and systems for treating nerve structures**

(30) Priority: 12.02.2014 US 201461938726 P
(71) Applicant: Perseus-Biomed Inc., Orangeburg, NY 10962 (US)
(72) Inventor: Behar, Boaz, 5591123 Ganei-Tikva (IL); Hertzberg, Yoni, 7311500 Moshav Ben-Shemen (IL); Henley, Avishai, 4531033 Hod-HaSharon (IL)
(74) Representative: Dennemeyer & Associates S.A.

(57) **Abstract**

There is provided a method of treating a patient comprising: screening a patient suffering from signs and/or symptoms of at least one medical condition secondary to increased sympathetic tone; selecting the patient for treatment by ablation of at least one neural structure containing sympathetic nerves to reduce the sympathetic tone, the neural structure comprises one or more of: celiac ganglion, superior mesenteric ganglion, inferior mesenteric ganglion, aorticorenal ganglion; selecting a target of at least one measureable parameter affected by the sympathetic tone; ablating at least a portion of the neural structure to deactivate the portion of the neural structure; monitoring changes in the at least one measureable parameter; comparing the at least one measureable parameter to the selected target; and performing another ablation of the same neural structure, and/or another neural structure, based on the comparison.

## Description

### FIELD AND BACKGROUND OF THE PRESENT INVENTION

The present invention, in some embodiments thereof, relates to systems and methods for treating nerve structures and, more particularly, but not exclusively, to systems and methods for ablating nerve structures to reduce sympathetic tone.

The sympathetic nervous system affects multiple activities in the human body. An increase in sympathetic tone may be associated with illnesses of the body. For example, in certain cases, increased sympathetic tone may be associated with hypertension (which is a major cause of illness worldwide).

Renal nerves are a small branch of the sympathetic nervous system. Renal denervation is a medical procedure where the sympathetic nerves lining the renal arteries are deactivated. Renal denervation may be effective in reducing blood pressure in refractory hypertensive patients.

Studies conducted in the 1940's in which most of the sympathetic nerves were removed by physically cutting them out from the body, in a procedure named sympathectomy, may have yielded a reduction in sympathetic tone, possibly without causing severe adverse events. However, the operative mortality rate of 4%, long recovery time, and/or complexity of the sympathectomy operation may not be tolerable for today's patients and surgeons.

Studies conducted with deactivation of the carotid body in animals suggest a possible greater effect of blood pressure reduction than that of renal denervation. However, treating the carotid body has some major drawbacks in its safety profile, as the carotid body is located next to the carotid artery directly leading to the brain, which may lead to stroke or other neurological damage.

Applicant's PCT Applications may provide additional background to some embodiments of inventions described below:
PCT/IB2010/056116 filed December 29, 2010;
PCT/IB2010/056117 filed December 29, 2010;
PCT/IB2012/054524 filed on September 2, 2012;
PCT/IB2012/054525 filed on September 2, 2012;
PCT/IB2013/060771 filed on December 10, 2012;
PCT/IB2013/061457 filed on December 31, 2013;
PCT/IB2013/061435 filed on December 31, 2013;

All these PCT applications are incorporated herein by reference in their entirety.

### SUMMARY OF THE PRESENT INVENTION

An aspect of some embodiments of the present invention relates to ablation of nerve structures to reduce sympathetic tone by monitoring one or more measurable parameters affected by the treatment, and repeating nerve structure ablation treatment based on the measured parameters. Optionally, the nerve structures that are ablated include one or more of: celiac ganglion, inferior mesenteric ganglion, superior mesenteric ganglion, aorticorenal ganglion, entirely or portions thereof, and/or two or more ganglia (for applicable ganglion type).

An aspect of some embodiments of the present invention relates to positioning an ablation device within the aorta and/or aorta branch vessels to geometrically encompass a target nerve structure, and applying energy to ablate the nerve structure within the encompassed region without damaging the aorta, aorta branch vessels and/or nearby tissue structures. Optionally, one or multiple electrodes are positioned within the aorta and/or branch vessel. Optionally, the aorta branch vessels are one or more of: celiac artery, branches of the celiac artery (left gastric artery, common hepatic artery, splenic artery), inferior mesenteric artery, superior mesenteric artery, renal artery, and/or one or more of the artery types (if applicable).

According to an aspect of some embodiments of the present invention there is provided a method of treating a patient suffering from a disease secondary to increased sympathetic tone, the method comprising: screening a patient suffering from at least one of signs and symptoms of at least one medical condition secondary to increased sympathetic tone; selecting the patient for treatment by ablation of at least a portion of at least one neural structure containing sympathetic nerves to reduce the sympathetic tone, the at least one neural structure comprises one or more of: celiac ganglion, superior mesenteric ganglion, inferior mesenteric ganglion, aorticorenal ganglion; selecting a target of at least one measureable parameter affected by the sympathetic tone; ablating at least a portion of the at least one neural structure to deactivate the portion of the at least one neural structure; monitoring changes in the at least one measureable parameter; comparing the at least one measureable parameter to the selected target; and performing another ablation of one or both of: the same at least one neural structure, and another at least one neural structure, based on the comparison.

According to some embodiments of the invention, the at least one medical condition secondary to increased sympathetic tone is overweight or obesity.

According to some embodiments of the invention, the at least one medical condition secondary to increased sympathetic tone is one or more of: type 1 diabetes, type 2 diabetes, prediabetes.

According to some embodiments of the invention, the at least one medical condition secondary to increased sympathetic tone is metabolic syndrome.

According to some embodiments of the invention, screening comprises screening the patient for at least two medical conditions. Optionally, the at least two medical conditions are obesity and hypertension.

According to some embodiments of the invention, the at least one neural structure contains efferent nerves.

According to some embodiments of the invention, the at least one neural structure contains gastric nerves.

According to some embodiments of the invention, selecting the patient comprises one or more of: selecting to reduce high blood pressure, selecting to reduce weight, selecting to increase insulin uptake.

According to some embodiments of the invention, selecting the patient comprises selecting the patient having enough weight before treatment to prevent or reduce the likelihood of patient being underweight after treatment.

According to some embodiments of the invention, selecting the patient comprises selecting the patient having at least normotensive blood pressure and normal weight.

According to some embodiments of the invention, ablation comprises ablating by one or more of: arterial embolization, thermal ablation, radiofrequency (RF) ablation, high intensity focused ultrasound (HIFU) ablation, drug injection, microwave ablation, electrical ablation.

According to some embodiments of the invention, the at least one measureable parameter is one or more of: weight of the patient, BMI of the patient, blood pressure of the patient, blood Lipin level, blood Grehline level, HbA1c, fasting plasma glucose (FPG) level, oral glucose tolerance test (OGTT) level.

According to some embodiments of the invention, performing another ablation further comprises performing another ablation until a predefined allowable number of treatments is reached or a predefined medical condition is met.

According to some embodiments of the invention, the predefined allowable number of treatments is three.

According to some embodiments of the invention, the ablating is performed in several predefined stages.

According to some embodiments of the invention, selecting the target comprises selecting the target by estimating treatment results so as to prevent or reduce the patient from overtreatment. Optionally, overtreatment comprises one or more of: underweight, hypotensive, hypoglycemic, hyperglycemic.

According to some embodiments of the invention, the at least one neural structure has dimensions at least about 2 mm X about 10 mm.

According to some embodiments of the invention, a time period between ablation sessions is at least 4 weeks.

According to some embodiments of the invention, the method further comprises performing a renal denervation procedure before and/or after ablation of the at least one neural structure.

According to some embodiments of the invention, the method further comprises: inserting a catheter into a lumen of the patient in proximity to the at least one neural structure, the catheter containing at least one sensor at a distal end region thereof for identifying parameters at the location of the catheter distal end region; identifying the parameters of the at least one sensor; and guiding an energy beam to the at least one neural structure based on the identified parameters of the at least one sensor, wherein the energy beam ablates the portion of the at least one neural structure. Optionally, the at least one sensor is a location sensor for determining a location within the patient. Optionally, the at least one sensor is a plurality of sensors of at least one type. Optionally, the at least one type is one or more of: temperature sensor, magnetic sensor, location sensor, ultrasound sensor, electromagnetic sensor, X-Ray sensor, microwave sensor, pressure sensor, and electrical impedance sensor. Optionally, the energy beam is one or more of: microwave, x-ray, HIFU, low intensity focused ultrasound (LIFU), sonoporation. Optionally, the energy beam is generated by a source external to the patient. Optionally, the lumen is located in proximity to the at least one neural structure. Optionally, the method further comprises transmitting energy towards the at least one sensor from a transmitter to identify energy parameters of the transmission by the sensor at the catheter distal end region within the body of the patient. Optionally, the method further comprises dynamically adjusting at least one of the parameters of the guided energy beam in response to tracked motion of the lumen, to direct the energy beam to ablate the portion of the at least one neural structure during motion of the lumen. Optionally, the method further comprises maintaining the position of the distal end region of the catheter inside the lumen. Optionally, the method further comprises generating one or more anatomical images of a body region containing the sensor, correlating the generated images with the identified location of the sensor, and guiding the energy beam to the neural structure based on the correlated image. Optionally, the images are generated in real time.

According to an aspect of some embodiments of the present invention there is provided a method of ablating a neural structure comprising: positioning at least one first electrode of a plurality of electrodes within an aorta; positioning another at least one second electrode of the plurality of electrodes within at least one aorta branch vessel; wherein the plurality of electrodes are positioned to encompass at least one neural structure located far from the positioned electrodes, wherein the at least one neural structure is located at least one of along and in proximity to an axis between the first and second electrodes, the first electrode positioned at one end of the axis, the second electrode positioned at an opposite end of the axis; and applying electrical energy to at least two the first and second of the plurality of electrodes forming the axis, the electrical energy applied in an amount to ablate at least a portion of the at least one neural structure without significantly ablating tissue surrounding the at least one neural structure.

According to some embodiments of the invention, the at least one neural structure comprises one or more of: celiac ganglion, superior mesenteric ganglion, inferior mesenteric ganglion, and aorticorenal ganglion.

According to some embodiments of the invention, the aorta branch vessel comprises at least one of: celiac trunk, common hepatic artery, left gastric artery, splenic artery, superior mesenteric artery, renal artery, and inferior mesenteric artery.

According to some embodiments of the invention, the neural structure or portions thereof are located far from the positioned electrodes comprises at least about 5 mm from the positioned electrodes.

According to some embodiments of the invention, the method further comprises applying the plurality of electrodes against an inner wall of the aorta and/or at least one aorta branch vessel.

According to some embodiments of the invention, the method further comprises selecting the at least two of the plurality of electrodes forming the axis from the plurality of electrodes, and applying energy to the selected electrodes.

According to some embodiments of the invention, the first electrode is positioned within the aorta proximally to a branch junction, and the second electrode is positioned within the at least one branch vessel distally to the branch junction.

According to some embodiments of the invention, applying electrical energy comprises alternating application of electrical energy to different electrodes at duration and amplitude to selectively damage the at least one neural structure over surrounding tissues when energy is applied to a single electrode, wherein the energy applied to the different electrodes ablates at least a portion of the neural structure.

According to an aspect of some embodiments of the present invention there is provided a catheter for ablation of a neural structure comprising: at least one rod sized for insertion into at least one of an aorta and an aorta branch vessel; a plurality of electrodes located at a distal end portion of the at least one rod, the electrodes disposed in a spaced apart arrangement along the distal end portion, the spacing selected to position at least one of a first of the plurality of electrodes within the aorta or aorta branch vessel when at least one of a second of the plurality of electrodes is positioned within another aorta branch vessel, the spaced apart arrangement selected to encompass at least one neural structure by forming an axis between the at least one first positioned electrode and at least one second positioned electrodes of the plurality of electrodes; and a controller programmed to apply electrical energy to at least the two of the plurality of electrodes forming the axis, the electrical energy applied in an amount to ablate at least a portion of the at least one neural structure located at least one of along and in proximity to the axis, without significantly ablating tissue surrounding the at least one neural structure.

According to some embodiments of the invention, the at least one neural structure comprises one or more of: celiac ganglion, superior mesenteric ganglion, inferior mesenteric ganglion, aorticorenal ganglion.

According to some embodiments of the invention, the at least one aorta branch vessel comprises at least one of: aorta, celiac trunk, common hepatic artery, left gastric artery, splenic artery, superior mesenteric artery, inferior mesenteric artery.

According to some embodiments of the invention, in proximity to the axis comprises less than about 50 mm from the axis.

According to some embodiments of the invention, the catheter further comprises at least one positioning element for one or both of securing the position of the electrodes and urging the plurality of electrodes against an inner wall of aorta and/or aorta branch vessel.

According to some embodiments of the invention, the controller is further programmed to select the at least two of the plurality of electrodes forming an axis from the plurality of electrodes, and applying energy to the selected electrodes.

According to some embodiments of the invention, the curvature of the electrodes is designed to contact the inner wall of the aorta and/or aorta branch vessel.

According to some embodiments of the invention, one or more electrodes are located on each of a plurality of rods, wherein the different rods are designed for positioning inside different aorta branch vessels.

According to some embodiments of the invention, the radial positions of the electrodes along the rod are selected for the electrodes to generally face the at least one neural structure.

According to some embodiments of the invention, the controller is programmed to deliver bipolar RF energy between the electrodes forming the axis.

According to an aspect of some embodiments of the present invention there is provided a catheter system for ablation of a neural structure comprising: at least two catheters comprising at least two rods from a common shaft or at least two independent rods; at least one of the catheters sized for insertion into an aorta or aorta branch vessel, and another of the at least two catheters sized for insertion into another aorta branch vessel; a plurality of electrodes located at distal end portions of the rods, the electrodes disposed in a predefined arrangement along the distal end portions, the predefined arrangement selected to encompass at least one neural structure by forming at least one axis between the electrode of the first catheter electrode, and the at least one another electrode of the aorta branch vessel when in the aorta branch vessel; and a controller programmed to apply electrical energy to at least the two of the plurality of electrodes of the at least two catheters forming the axis, the electrical energy applied in an amount to ablate at least a portion of the neural structure located at least one of along and in proximity to the axis, without significantly ablating tissue surrounding the neural structure.

According to some embodiments of the invention, the at least one neural structure comprises one or more of: celiac ganglion, superior mesenteric ganglion, inferior mesenteric ganglion, aorticorenal ganglion.

According to some embodiments of the invention, the one aorta branch vessel comprises at least one of: aorta, celiac trunk, common hepatic artery, left gastric artery, splenic artery, superior mesenteric artery, inferior mesenteric artery.

According to an aspect of some embodiments of the present invention there is provided a method for ablating a neural structure located away from a wall of a body lumen comprising: positioning an ablation element within at least one of an aorta and aorta branch vessel, for directing energy at a focal region containing at least one neural structure at least 5 mm away from a surface of the at least one of the aorta and aorta branch vessel, the ablation element located at a distal region of a catheter; and expanding a positioning element with at least one lumen for allowing blood to flow through the at least one lumen when the positioning element is expanded inside a blood vessel, the ablation element located within the positioning element, the positioning element having an expanded size and shape selected for securing the position of the ablation element inside the blood vessel when the positioning element is in an expanded state, the positioning element located at the distal region of the catheter; wherein the positioning element is filled with fluid suitable for transmission of the directed energy to the inner wall of at least one of the aorta and aorta branch vessel.

According to some embodiments of the invention, the at least one neural structure comprises one or more of: celiac ganglion, superior mesenteric ganglion, inferior mesenteric ganglion, aorticorenal ganglion.

According to some embodiments of the invention, the positioning element is an inflatable balloon, and further comprising inflating the inflatable balloon.

According to some embodiments of the invention, the directed energy is selected from the group: high intensity focused ultrasound, low intensity focused ultrasound, microwave, X-ray.

According to some embodiments of the invention, the ablation element is a transmitting element selected from the group: phased array transmitter, single element transmitter, annular array transmitter, reflector.

According to some embodiments of the invention, the method further comprises adjusting the distance of the focal region of the ablation element to different distances to ablate the at least one neural structure.

According to some embodiments of the invention, the ablation element is positioned in the esophagus.

According to some embodiments of the invention, the method further comprises adjusting the energy to ablate the at least one neural structure.

According to some embodiments of the invention, the method further comprises adjusting an energy focus position during treatment to different distances from the lumen, the distances ranging from about 5 mm to about 50 mm.

According to some embodiments of the invention, the focal region is between about 5 mm and about 50 mm from the ablation element.

According to some embodiments of the invention, the size of the focal region is between about 5 mm and 50 mm.

According to some embodiments of the invention, the method further comprises steering the directed energy to ablate the at least one neural structure.

According to an aspect of some embodiments of the present invention there is provided a catheter for ablation of a neural structure comprising: a catheter sized for insertion into at least one of an aorta and aorta branch vessel; a plurality of needles deployable to a length of about 10 mm, the needles designed to ablate a target neural structure; a structure for housing the plurality of needles, the structure disposed on a distal end region of the catheter; and a releasing mechanism for controlling the release of the needles from the structure.

According to some embodiments of the invention, the needles are deployable to a length of about 20 mm.

According to some embodiments of the invention, at least one of the needles have a plurality of holes along a shaft for releasing an ablation substance.

According to some embodiments of the invention, the releasing mechanism controls deployment of the needles to different lengths.

According to some embodiments of the invention, the needles are designed for cryo-ablation or RF ablation.

According to some embodiments of the invention, the needles are positioned to inject a wall of a blood vessel at no more than an arc length of about 30 degrees around the circumference of the blood vessel.

According to an aspect of some embodiments of the present invention there is provided a method for ablating a neural structure located away from a wall of a body lumen comprising: positioning a ultrasound transmitter within an aorta branch vessel, for directing energy at a focal region containing at least one neural structure at least 5 mm away from a surface of the aorta branch vessel, the ultrasound transmitter located at a distal region of a catheter; and expanding a positioning element, the ultrasound transmitter located within the positioning element, the positioning element having an expanded size and shape selected for securing the position of the ultrasound transmitter inside the aorta branch vessel when the positioning element is in an expanded state, the positioning element located at the distal region of the catheter; and adjusting an ultrasound energy beam to ablate the target neural structure, wherein the ultrasound energy beam is transmitted through a part of the circumference of the aorta branch vessel having an arc length of no more than about 45 degrees; wherein the positioning element is filled with fluid suitable for transmission of the directed energy to the inner wall of at least one of the aorta and aorta branch vessel.

According to an aspect of some embodiments of the present invention there is provided a catheter for ablating a neural structure located away from a wall of a body lumen comprising: a catheter sized for insertion into a blood vessel and sized for positioning within an aorta branch blood vessel; a transmitter disposed at a distal region of a catheter; and an expandable positioning element containing a reflector and the transmitter, the reflector having an expanded size and shape for reflecting energy transmitted by the transmitter at a focal region containing at least one neural structure at least 5 mm away from a surface of the aorta branch vessel, the reflector coupled to the expandable positioning element so that expansion of the positioning element expands the reflector; wherein the positioning element has an expanded size and shape for securing the position of the transmitter and reflector inside the aorta branch vessel when the positioning element is in an expanded state, the positioning element located at the distal region of the catheter; wherein the positioning element is filled with fluid suitable for transmission of focused energy to the inner wall of at least one of the aorta and aorta branch vessel.

According to some embodiments of the invention, the distal end of the catheter is sized for insertion into the aorta branch vessel when the positioning element and the reflector are collapsed, and the diameter of the expanded reflector is larger than the diameter of the aorta branch vessel when expanded.

According to some embodiments of the invention, the transmitter is aligned at one side of the expanded positioning element, or located approximately in the middle of the expanded positioning element, and the transmitter is oriented to transmit in the opposite direction of the target neural structure, towards the reflector.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a schematic illustration of an aorta and aorta branch vessels with nearby ganglia, in accordance with some embodiments of the present invention;
FIG. 2 is a flowchart of a method of treating a patient with increased sympathetic tone, in accordance with some embodiments of the present invention;
FIG. 3 is a flowchart of a method of ablating a nerve structure based on the method of FIG. 2, in accordance with some embodiments of the present invention;
FIG. 4 is a schematic illustration of some systems for nerve ablation in a catheterization laboratory, in accordance with some embodiments of the present invention;
FIG. 5 is a flowchart of a method of treating an obese patient, in accordance with some embodiments of the present invention;
FIG. 6 is a flowchart of a method of treating a hypertensive patient, in accordance with some embodiments of the present invention;
FIG. 7 is a schematic block diagram of a system for ablation of nerve structures from within the aorta and/or aorta branch vessels, in accordance with some embodiments of the present invention;
FIG. 8 is a flowchart of a method of ablating one or more neural structures by energy delivery through electrodes, in accordance with some embodiments of the present invention;
FIG. 9 is a schematic illustration of a catheter design for ablation of nerve structures, in accordance with some embodiments of the present invention;
FIG. 10 is an illustration of the catheter of FIG. 9, with one or more positioning elements, in accordance with some embodiments of the present invention;
FIG. 11 is an illustration of two catheters for ablation of nerve structures, in accordance with some embodiments of the present invention;
FIG. 12 is yet another schematic illustration of a catheter design for ablation of nerve structures, in accordance with some embodiments of the present invention;
FIG. 13 is yet another schematic illustration of a catheter design for ablation of nerve structures, in accordance with some embodiments of the present invention;
FIG. 14 is yet another schematic illustration of a catheter design for ablation of nerve structures, in accordance with some embodiments of the present invention;
FIG. 15 is yet another schematic illustration of a catheter design for ablation of nerve structures, in accordance with some embodiments of the present invention; and
FIG. 16 is yet another schematic illustration of a catheter design for ablation of nerve structures, in accordance with some embodiments of the present invention.

### DESCRIPTION OF SPECIFIC EMBODIMENTS OF THE PRESENT INVENTION

The present invention, in some embodiments thereof, relates to systems and methods for treating nerve structures and, more particularly, but not exclusively, to systems and methods for ablating nerve structures to reduce sympathetic tone.

An aspect of some embodiments of the present invention relates to treatment of a patient suffering from increased sympathetic tone, by ablation of nerve structures, based on monitoring levels of one or more measureable parameters that are affected by the increased sympathetic tone. Optionally, nerve structure ablation treatments are repeated based on the monitored levels, for example, after at least 2 weeks, at least 4 weeks, at least 8 weeks, or other time intervals. In this manner, the patient may be treated with customized amount of ablation to achieve desired results. Splitting the treatment up into multiple treatment sessions may be safer, for example, facilitating the appliance of less energy to surrounding tissues during the ablation, reducing the risk of damaging surrounding tissues (e.g., aorta). Overtreatment and/or sudden homeostatic fluctuations may be reduced and/or prevented by performing the multiple sessions with monitoring of the effects.

Optionally, the nerve structures which are ablated include one or more of: celiac ganglion, inferior mesenteric ganglion, superior mesenteric ganglion, aorticorenal ganglion, entirely or portions thereof, and/or two or more ganglia (for applicable ganglion type).

Optionally, ablation of the nerve structure (e.g., ganglia) is performed by energy delivery from within major blood vessels. Alternatively or additionally, ablation of the nerve structure is performed by energy delivery from outside the body of the patient, for example, as described with reference to Applicant's PCT Application IB2012/054524, and/or Applicant's PCT Application IB2012/054525, and/or other Applicant's Applications which were mentioned in the Background section above. Optionally, the blood vessels are located in proximity to the target ganglia. For example, energy is delivered from one or more of: the aorta, celiac artery, branches of the celiac artery (left gastric artery, common hepatic artery, splenic artery), inferior mesenteric artery, superior mesenteric artery, renal artery, and/or one or more of the artery types (if applicable), and/or other branch vessels of the aorta, and/or other body lumens (e.g., veins, esophagus, stomach).

Optionally, multiple treatment sessions are preselected. Optionally, treatment sessions are preselected to treat a large nerve structure, by partial treatments during each treatment session. The nerve structure may not be suitable for treatment within a single session. For example, energy may not be able to be applied to treat the entire nerve structure within a single session without damaging surrounding tissues and/or without damaging the aorta and/or aorta branch vessels. In such a case, parts of the nerve structure may be ablated during each treatment, for example, depending on the ability of the surrounding tissue to absorb energy without significant damage. In one example, comparing the celiac, superior mesenteric, inferior mesenteric and/or aorticorenal ganglia to renal nerves, the ganglia are much larger in size and located further away from the inner wall of large blood vessels (e.g., aorta, celiac trunk, superior, interior mesenteric arteries, renal arteries), and therefore may require multiple treatment sessions. Several treatments may be planned in advanced (and/or decided upon during follow up) to ablate the desired volume and/or number of ganglia without damaging the large blood vessels and/or other significant surrounding tissues.

Ablation of nerves within the arterial wall and/or close to the arterial wall, such as for renal artery nerves, may be performed, for example, with radiofrequency (RF) catheters, ultrasound catheters, drug injection catheters, catheters guiding external ultrasound around the vessel, x-rays, microwaves and/or other methods and/or systems. Ablation of ganglia (e.g., celiac, superior mesenteric, inferior mesenteric, aorticorenal) and/or other nerve structures may be different than ablation of renal nerves and/or other nerves within and/or close to the arterial wall. For example, the ganglion may be a mass of neural substance, rather than a set of small string-like nerves. The ganglion may reach a size of about 3 centimeters in length and about 3-5 mm in width. The large size of the ganglion may make it difficult to ablate relative to renal nerves, especially without damaging surrounding tissue structures such as the arterial wall.

Ganglia maybe located far from the lumen of the blood vessel, for example, more than about 1mm, or 3 mm, or 5 mm, or 7 mm, or 10 mm, or 20 mm, or 30 mm, or 50 mm, or other distances. Optionally, the distance is overcome by positioning electrodes within nearby blood vessels, to encompass the target neural structure with the multiple electrodes. Energy application from the encompassing electrodes is controlled to selectively ablate the far neural structure without damaging other tissues between the electrodes and the target neural structure. Alternatively or additionally, the distance is overcome by applying energy that is focused on the target neural structure. The focused energy may be generated by a transmitter located within nearby blood vessels of the body, or outside the body of the patient.

Ganglia may be surrounded by tissues that limit heat transfer from the artery wall (e.g., loosely connected with connective tissue), which may pose a difficulty in ablation. Ganglia located in proximity to the aorta pose a challenge in ablation from within the aorta and/or from within branches of the aorta. The aorta is a large blood vessel that feeds more of the tissues of the body, having a relatively high blood flow and/or blood pressure. Damage to the aortic wall (e.g., from traditional RF catheters) may cause an aneurysm and/or blood leaks, which may be fatal to the patient. Furthermore, blood flow within the aorta is difficult to block, and/or any such blockage may not be tolerated by end organs received blood. In view of the above, methods and/or systems designed for ablation of nerves within and/or next to blood vessel walls (e.g., renal nerves) may not be suitable for ablation of ganglia (e.g., celiac, superior mesenteric, inferior mesenteric, aorticorenal), especially from within the aorta and/or branches of the aorta.

Optionally, ablation treatment is performed from within the larger blood vessels (e.g., aorta and/or aorta branch vessels). Optionally, the treatment is planned based on the ability of the large blood vessels to absorb energy without being significantly damaged, when ablation energy is delivered from the large blood vessel to the nerve structure. Each treatment session may include ablating a portion of the target ganglion, with the total target portion ablated after several treatments.

Optionally, the parameter measurements are repeated and monitored to prevent or reduce over-treatment effects. Optionally, treatment is not continued when the measurable parameter levels indicate sufficient treatment.

Optionally, the increased sympathetic tone is related to a medical condition, for example, one or more of: hypertension, resistant hypertension, insulin resistance, type 1 diabetes, type 2 diabetes, prediabetes, metabolic syndrome, obesity, and/or overweight.

Optionally, ablation is performed by an extracorporeal high intensity focused ultrasound (HIFU) system. Alternatively or additionally, ablation is performed by using a sensor within the patient to position and/or aim an energy projection device, for example, as taught in Applicant's PCT Application PCT/IB2012/054524, and Applicant's PCT Application PCT/IB2012/054525, and/or other Applicant's Applications which were mentioned in the Background section above. The aiming of the HIFU beam towards the target nerve structure may be based on the methods described in Applicant's PCT Applications.

Optionally, the ablation is performed to achieve a weight loss target in the patient. Alternatively or additionally, the ablation is performed to achieve a target reduction in blood pressure. Optionally, patients selected for treatment suffer from both high blood pressure and excess weight.

An aspect of some embodiments of the present invention relates to ablation of one or more neural structures (e.g., ganglia) by geometrically encompassing electrodes placed within the aorta and/or aorta branch vessels, without significantly damaging the vessels and/or other tissues. Alternatively or additionally, the method is performed using other energy delivery devices from within the aorta and/or branch vessels. The aorta branch vessel is, for example, a celiac trunk, a common hepatic artery, a left gastric artery, a splenic artery, superior mesenteric artery, renal artery, and/or inferior mesenteric artery. The methods and/or systems of ablation of nerve tissue from within the aorta and/or aorta branch vessels may be safe, reducing and/or preventing damage to the aorta and/or aorta branch vessels.

Optionally, one or more electrodes are positioned within the aorta, and one or more electrodes are positioned within a branch of the aorta. Alternatively or additionally, one or more electrodes are positioned within one aorta branch, and one or more electrodes are positioned in other aorta branch vessels (and/or celiac artery branch vessels).

Optionally, the electrodes form one or more axes between them. Optionally, one or more electrodes are positioned at one end of the axis, and the other electrodes are positioned at the other end of the axis.

Optionally, the neural structure is selectively ablated over surrounding tissue. The blood vessel wall may not suffer damage, preventing or reducing leaks and/or aneurysms. Optionally, the ablation is performed over multiple treatment sessions.

Optionally, the neural structures lies far from the inner wall of the aorta and/or branch vessels, for example, at least about 5 millimeters (mm) away, or at least about 10 mm, or at least about 20 mm, or at least about 30 mm, or at least about 50 mm, or other smaller, intermediate or larger distances away.

Optionally, the electrodes are positioned against the inner wall of the aorta and/or branch vessel.

Optionally, the neural structure lies along an axis drawn between any two electrodes. Alternatively or additionally, the neural structures lies in proximity to the axis, for example, within about 1 mm, or about 3 mm, or about 5 mm or about 10 mm, or about 20 mm, or about 50 mm, or other smaller, intermediate or larger distances. Optionally, the electrodes are spaced apart with respect to the neural structure. Electrical conduction to the nerve structure may occur, for example, through intermediate tissue and/or through other nerve structures, for example, axons connecting to the nerve structure.

Alternatively or additionally, multiple electrodes are arranged in the anatomy around the neural structure to form a partial outline of a virtual box, dome, sphere, and/or other full and/or partial geometrical shape arrangements. Electrodes may be activated together and/or in sequence. The nerve structure may be trapped within the geometrical arrangement, absorbing the electrical energy.

Optionally, energy is delivered by the electrodes to selectively ablate the neural structures without significant damage to nearby tissues. For example, important structures such as major arteries are not damaged (e.g., to leak and/or form an aneurysm). Some damage to nearby structures may be allowed, for example, to nearby muscle and/or connective tissue.

An aspect of some embodiments of the present invention relates to a catheter with a plurality of microneedles for ablation of a target nerve structure located far from the inner vessel wall. The needles may inject a chemical substance for ablation. The microneedles may reach far into the tissue, to ablate the target nerve structure at one or more locations. The length of the microneedle may be adjustable. Injection may be performed at different depths within the tissue, by varying the length of the needle, and/or through multiple ports along the shaft of the needle. Optionally, injection is targeted to a defined location of the inner vessel wall to reach the target tissue.

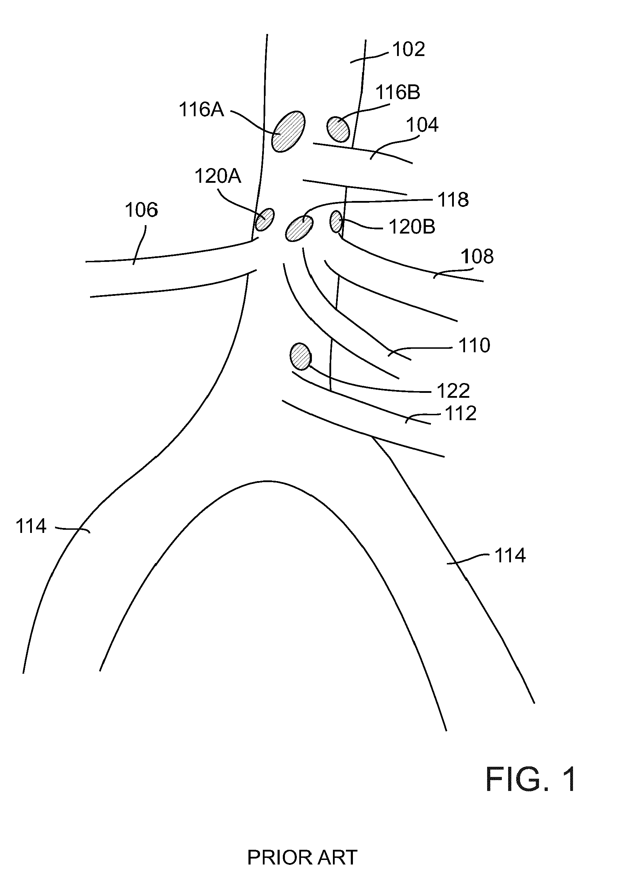

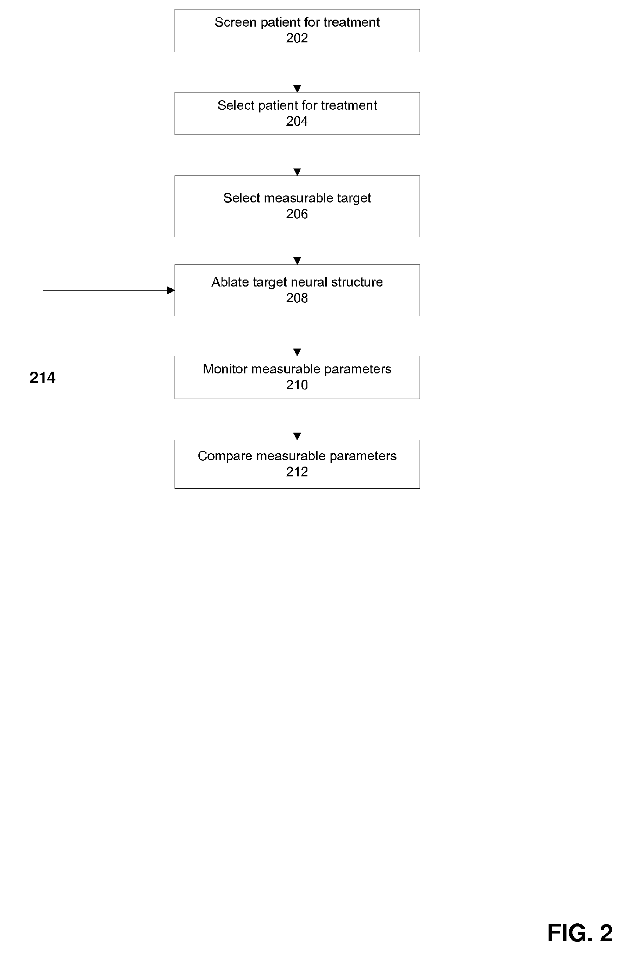

For purposes of better understanding some embodiments of the present invention, as illustrated in FIGs. 2-14 of the drawings, reference is first made to the anatomy of a patient as illustrated in FIG. 1, showing locations of possible nerve structure treatment targets, such as ganglia. An abdominal aorta 102 is schematically illustrated, as would be seen when facing the patient. Aorta 102 may contain one or more branching vessels: celiac artery (or trunk) 104 (which may divide into a common hepatic artery, left gastric artery and splenic artery), right renal artery 106, left renal artery 108, superior mesenteric artery 110, inferior mesenteric artery 112, and iliac arteries 114.

One or more nerve structures (such as ganglia) may be located in proximity to the arteries: celiac ganglia 116A-B in proximity to celiac artery 104, superior mesenteric ganglion 118 in proximity to superior mesenteric artery 110, aorticorenal ganglia 120A-B in proximity to renal arteries 106 and 108, inferior mesenteric ganglion 122 in proximity to interior mesenteric artery 112.

It is noted that the anatomical structures illustrates in FIG. 1 are exemplary. Patients may vary in anatomy, for example, different numbers, different sizes, relative locations, and/or other parameters of branch vessels, ganglia, and/or other nerve structures.

As described herein, the term *ganglion* may sometimes refer to a target nerve structure for ablation. The term *ganglion* may sometimes refer to a ganglion of the sympathetic nervous system. The term *ganglion* may sometimes refer to a structure in which nerves synapse with one another. The term *ganglion* may also sometimes refer to other nerve structures, such as nerve bodies, axons, or other nerve structures. The term *ganglion* may sometimes be inclusive, meaning trunk (e.g., sympathetic trunk), nerve plexus, and/or other anatomical terms denoting nerve structures. For example, ablation of the ganglion may sometimes mean ablation of one or both of the ganglia, ablation of nearby plexus, and/or ablation of other nerve structures which may broadly be defined as the ganglion, for example, based on an anatomical atlas, imaging methods, physician experience, visualization of the nerve structures, or other identification methods.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Inventors discovered that there may be a greater effect of reducing sympathetic tone, and possibly greater effect in reducing blood pressure, increasing insulin uptake and/or other sympathetic tone related conditions, by treating larger sections of the sympathetic nervous system, for example, as compared to treating renal nerves. Treatment of efferent and/or afferent components of the sympathetic nervous system may reduce over-activity of end organs. For example, over-activity of the kidney as measured by norepinephrine spillover may be reduced by renal denervation. Treatment of parts of the sympathetic nervous system may achieve several beneficial effects at once. For example, deactivation of renal nerves may decrease insulin resistance in patients with refractory hypertension. This may be due to a decrease in sympathetic activity leading to the gastric system.

Obesity and diabetes may also be linked to increased sympathetic tone. In diabetic patients, reduction of sympathetic tone may be associated with increased insulin uptake. Increased sympathetic tone may also be associated with sleep apnea and other diseases.

Inventors realized that damage to certain nerve structures, in addition to possible weight loss, may also be associated with a systemic reduction in other blood chemicals (e.g., hormones) affected by increased sympathetic tone. For example, the gastric sympathetic nerves are connected to the central nervous system through the celiac ganglion, the superior mesenteric ganglion, the inferior mesenteric ganglion and/or the aorticorenal ganglion. The celiac ganglia, as well as the superior, inferior mesenteric ganglia and/or aorticorenal ganglia collect much more of the sympathetic activity than the renal nerves, and therefore are also likely to have a greater effect on hypertension.

Inventors discovered that deactivation of the celiac ganglia and/or the superior mesenteric ganglion and/or the inferior mesenteric ganglion and/or aorticorenal ganglia, in overweight and/or diabetic and/or hypertensive and/or metabolic syndrome individuals, and/or other sympathetic tone related illness may achieve a reduction of sympathetic tone, reduction of weight and/or increase in insulin uptake. Inventors discovered that denervation of other neural structures of the sympathetic neural system may also achieve similar results, for example, ablation of parts of the sympathetic chain, splanchnic nerves, nerve plexuses, or other nerve structures.

Inventors hypothesize that elevated activity of the sympathetic system affects the metabolic system, for example, affecting obesity, glucose uptake, insulin resistance, metabolic syndrome and/or other mechanisms related to diabetes and/or other conditions (e.g., Ghrelin, lipid, and/or other digestive mechanisms).

Inventors conducted animal experiments with adult pigs with two control groups; the treated group has received a celiac ganglion partial thermal ablation by the use of an extracorporeal HIFU transducer guided by an intravascular catheter with acoustic sensors. Fine aiming of a HIFU beam towards an intended target may be accomplished using feedback from an ultrasound sensor in a catheter, as taught in PCT Applications PCT/IB2012/054524, PCT/IB2012/054525, and/or other cited applications. The ablations of portions of the celiac ganglion were validated by histology examination of the tissue of the treated animals. The first control group did not receive any treatment. The second control group received a bilateral renal nerve thermal ablation by the use of the same extracorporeal HIFU transducer guided by the same type of intravascular catheter with acoustic sensors (ablations were validated by histology). Both control groups gained weight (as normal in adult pigs) of an average of 4.9 kilograms (Kg) in four weeks, with all pigs gaining at least 3 Kg. All treatment pigs have gained an average of about 0 Kg.

The methods and systems described herein may overcome adverse events related to other treatment methods of deactivation ganglia, for example, conducted with needle aspiration either with a CT and/or fluoroscopic guided posterior approach, advancing the needles near the spinal cord, or using an ultrasound guided intra esophageal approach puncturing the esophagus and reaching the ganglion from there. The posterior needle aspiration approach may damage the spine and cause paralysis or other neural damage. The trans-esophageal needle approach may cause infections by the contact between the esophagus (non sterile) and the ganglion (sterile). Some embodiments described herein relate to a non-puncturing approach which has accurate aiming, such as a remote energy transmission (extracorporeal and/or from inside the aorta and/or branch vessels). Some embodiments may not be associated with these type of adverse events and/or may reduce other risks, for example, damage to the aorta and/or branch vessels (e.g., blood leak, aneurysm formation) and/or other structures (e.g., central nervous system). Some embodiments may selectively damage nerve structures lying far away from the internal wall of the aorta and/or aorta branch vessels, even when energy is being delivered from within the aorta and/or aorta branch vessel itself.

Referring now to the drawings, FIG. 2 is a flowchart of a method of treating a patient suffering from one or more medical conditions secondary to increase sympathetic tone, in accordance with some embodiments of the present invention. The medical conditions are, for example, hypertension, resistant hypertension, insulin resistance, type 1 diabetes, type 2 diabetes, prediabetes, metabolic syndrome, obesity, overweight or other conditions. The method may allow for a customized treatment of the patient, while reducing and/or preventing overtreatment effects.

Optionally, at 202, a patient is screened for treatment based on the method. Optionally, the patient displays signs and/or symptoms of a medical condition. The signs and/or symptoms and/or the medical condition may at least be partially due to increased sympathetic tone.

Optionally, the patient is screened for having at least two medical conditions. Both medical conditions may be improved by the treatment.

Optionally, screening is performed, for example, based on blood pressure, body mass index (BMI), fasting plasma glucose levels, or other levels. For example, blood tests may suggest that the patient is diabetic, for example, HbA1c > 6.5%, random plasma > 11.1 millimoles per liter (mmol/l), fasting plasma glucose > 7.0 mmol/l, or other values. The diabetes related parameters may be used as measurement targets and/or monitored, for example, as described herein.

Optionally, at 204, the patient is selected for treatment by ablation of at least a portion of at least one neural structure containing sympathetic nerves to reduce the sympathetic tone and/or affect the end organ efferent sympathetic nerves are connected to.

The patient may be selected based on the ability to achieve a desired target result, for example, one or more of: reduction in blood pressure, reduction in weight, increase in insulin uptake, and/or other desired results.

The patient may be selected in view of the expected results of ablation of the nerve structure. The patient may be selected to prevent or reduce effects of over treatment. As the treatment may reduce both blood pressure and weight, the patient may be selected to have at least normotensive blood pressure (e.g., 120/80 mmHg) and/or at least normal weight (e.g., 18.5 BMI). In this manner, additional reductions in blood pressure and/or weight might not negatively impact the health of the patient.

Optionally, at 206, a target of at least one measurable parameter affected by the increased sympathetic tone is selected. Examples of measureable parameters include: weight of the patient, BMI of the patient, blood pressure of the patient, blood Lipin level, blood Grehline level, HbA1c, fasting plasma glucose (FPG) level, oral glucose tolerance test (OGTT) level, or other parameters.

The target may be selected based on estimated treatment results to prevent or reduce the patient from overtreatment, for example, to prevent or reduce the risk of becoming underweight, hypotensive, hypoglycemic, and/or hyperglycemic.

The target may be based, for example, on a table of experimental results, on the experience of the physician, on a mathematical equation, or other methods. Data may be obtained from treated patients, animal models, mathematical models, or other sources.

Optionally, a baseline is determined for the one measureable parameter. Optionally, a first set of measurements is taken to establish the baseline.

At 208, one or more neural structures are ablated. The ablation is performed to deactivate at least a portion of the nerve cells within the neural structure. The number of nerve cells that are ablated, and/or the volume of the nerve structure that is ablated, is, for example, about 10%-50%, about 25%-50%, about 25%-75%, about 50%-100%, about 50%-75%, about 75%-90%, or other ranges. Ablation may be permanent or temporary. Ablation may prevent the nerve cells from conducting signals.

The neural structure is, for example, the celiac ganglion, superior mesenteric ganglion, inferior mesenteric ganglion, and/or aorticorenal ganglion, and/or other nerve structures. The neural structure may contain efferent sympathetic nerves. The neural structure may contain gastric nerves.

The nerve structure may have dimensions of, for example, at least about 1 mm X 5 mm, or at least about 2 mm X 10 mm, or at least about 3 mm X 15 mm, or at least about 5 mm X 30 mm, or other dimensions.

Ablation may be performed, for example, by the systems and/or methods described herein, and/or by other suitable methods, for example, arterial embolization, thermal ablation, radio frequency (RF) ablation, HIFU ablation, drug injection, microwave catheter, or other methods.

Optionally at 210, measurements of the parameters are repeated. Optionally, values of the measurements are monitored for changes. Optionally, measurements are monitored to detect a decrease (or increase) in value over time. Optionally, measurements are monitored to detect stabilization at a certain value.

The frequency of repeating the measurements may depend on the measurement itself, for example, once a day, once a week, once a month, or other time frames.

The measurements may be monitored for changes, either relative to the baseline and/or for relative changes between treatments.

Optionally, at 212, the measured parameters are compared to the selected treatment target (e.g., block 206). Optionally, the comparison is performed after stabilization of the measured values.

Optionally, at 214, the ablation treatment (e.g., block 208) is repeated. Treatment may be repeated if the target has not been met. Treatment may be repeated, for example, after at least 2 weeks, 4 weeks, 8 weeks, or other time periods.

Optionally, the same nerve structure may be further ablated. Alternatively or additionally, one or more different nerve structures may be ablated.

Optionally, the treatment plan may include several ablation sessions from the outset of the treatment plan. For example, treatment may be in several stages. At each stage, different targets may be set to help determine when the next stage is performed. The different stages may help the body adjust to the new neural anatomy and may help prevent or reduce sudden homeostatic fluctuations.

Alternatively, treatment is stopped. Treatment may be stopped if the target has been met. Treatment may be stopped after a predefined allowable number of treatments have been reached, for example, at least 1, 2, 3, 4, 5, or more.

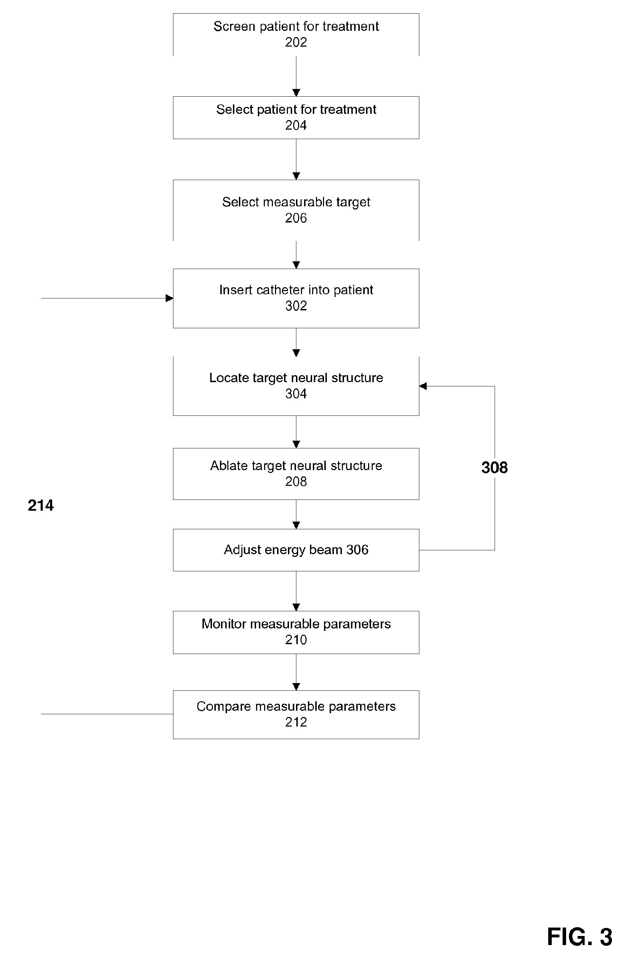

Reference is now made to FIG. 3, which is a flowchart of an exemplary method of ablation, based on the method of FIG. 2, and based on the method of ablation, for example, as described in PCT Applications IB2012/054524 and IB2012/054525, or other Applications by Applicant, which teach devices and methods for accurately finely aiming an energy beam using feedback from a beam-sensitive sensor positioned near a therapeutic target. Blocks 202-214 correspond to FIG. 2. Additional blocks are described.

Optionally, at 302, a catheter is inserted into a lumen of the patient in proximity to the target neural structure. The catheter may be positioned in the aorta, celiac artery (and/or branches thereof), super mesenteric artery, inferior mesenteric artery, renal artery, and/or other vessels, esophagus, urethra, nasal canal, throat, eye canal, and/or other lumens and/or body cavities. Optionally, the position of the catheter is secured within the vessel, for example, by using a positioning element as described herein.

Optionally, the catheter contains at least one location sensor at a distal end region thereof, for identifying the location of the catheter distal end region and/or shaft and/or other catheter portions, when the catheter is in position inside the blood vessel or lumen. Examples of location sensors include: magnetic location sensors, ultrasonic sensors, heat sensors, sensors for measuring transmitted energy, sensors for determining effects of transmitted energy on tissue, sensors for determining geometrical relationships between any two or more of: the sensor (and/or catheter), the target neural tissue, the transmitter, or other suitable sensors.

Optionally, sensors include one or more different types of sensors, for example: temperature sensor, magnetic sensor, location sensor, ultrasound sensor, electromagnetic sensor, X-Ray sensor, microwave sensor, pressure sensor, and/or electrical impedance sensor.

Optionally, each sensor or group of sensors is coupled to a positioning element for maintaining the position of the sensor relative to the body lumen. Examples of positioning elements include: expandable cages (e.g., made from flexible biocompatible plastic and/or nickel-titanium), a balloon, shaped portion of catheter shaft (e.g., into a helix), or other structures. Optionally, the position of the distal end region of the catheter is fixated within the lumen, for example, by expanding and/or deploying the positioning element inside the lumen. The positioning element may be arranged to contact the sensors with the vessel wall.

Alternatively or additionally, the catheter assists in targeting an external energy beam to the target neural structure. Optionally, the catheter contains one or more sensors to guide the external beam. Optionally, the sensors are energy sensitive sensors to identify required parameters for transmission of the energy beam. Alternatively or additionally, sensors are location sensors.

In one example, the catheter contains multiple sensors, at least one set close to the tip, and a second set of one or more additional sensors, proximally located relative to the first set of distal sensors. The sensors may be acoustic sensors. The external transmitter may be an ultrasound transmitter optionally located outside the body. To ablate, for example, the celiac ganglion (or portions thereof), the catheter is positioned with the distal end region inside the celiac artery, or branches thereof. Optionally, the positioning element is expanded to secure the position of the sensors at the distal end region within the celiac artery. Optionally, the second set of sensors is located in the aorta when the first set is located in the celiac artery. A second positioning element may be expanded to secure the position of the second set of sensors in the aorta. The first and second set of sensors may geometrically encompass the celiac ganglion.

Optionally, at 304, the location of the neural structure relative to the location sensor is determined.

Optionally, the location is identified by transmitting energy towards the location sensor from a transmitter, for example, from an external transmitter located inside the body (e.g., in the esophagus) and/or outside the body. Optionally, the energy is transmitted to cover the region between the transmitter and the sensors. Optionally, the target neural structure lies within the covered region.

Optionally, the received energy signals are analyzed to determine the location of the sensor relative to the transmitter. The transmitted energy may be low level energy that does not cause tissue damage (e.g., does not ablate). With the location of the sensor being identified, the location of the neural structure relative to the sensor may be estimated. Alternatively, the location of the neural structure relative to the sensor is determined, for example, based on acquiring an anatomical image of the sensor and neural structure, and correlating the anatomical image of the neural structure and sensor with the identified position of the sensor. The anatomical images may be generated in real time to direct the beam, for example, using fluoroscopic imaging. Optionally, anatomical images of the patient (e.g., CT and/or MRI) are before treatment, without taking into account the sensor's position (e.g., without the catheter in position). Treatment may be planned according to the acquired images, and/or correlated with additional images, for example, images that include the sensors in position (e.g., fluoroscopic images). The neural structure may not be visually apparent on images. The location of the neural structure may be estimated based on relative location to structures visible on images, for example, the location of the celiac ganglion may be estimated to be at the junction of the celiac artery and the aorta.

The remote transmitter may be, for example, an extracorporeal HIFU transducer, a trans-esophageal HIFU transducer, an X-ray transducer, or a transducer of other types of energy.

Optionally, at 208, ablation is performed by guiding an energy beam to the target neural structure based on the position of the location sensor. Optionally, the energy beam is one or more of: microwave, x-ray, other electromagnetic frequencies, HIFU, Low Intensity Focused Ultrasound (LIFU), sonoporation. Optionally, the energy beam is directed from a source external to the patient. Optionally, the beam is directed from a source internal to the patient at a different location than the sensors.

Optionally, the beam is directed towards the celiac ganglion (one or both), the superior mesenteric ganglion, the inferior mesenteric ganglion, the aorticorenal ganglion (one or both), or other ganglia. The beam may be directed to target the entire ganglion, or part of the ganglion. The beam may be directed to target nerve structures surrounding the ganglia, for example, nerve plexus. The beam may be directed to target two or more ganglia simultaneously, or at different times.

Optionally, at 306, the energy beam is adjusted, for example, the power, position, phase and/or other parameters. Optionally, the energy beam is dynamically adjusted in response to tracked motion of the position sensor (which denotes the motion of the lumen). Optionally, the energy beam is adjusted to direct the energy beam to ablate the portion of the neural structure during motion of the lumen.

Optionally, energy measuring sensors monitor the delivered ablation energy. Optionally, the sensors provide feedback signals for adjusting the applied energy level so that the energy beam has sufficient energy for ablation of the target nerve structure. Optionally, the energy beam hitting the sensor is adjusted to have enough energy for ablation. Alternatively or additionally, the energy beam hitting the target neural structure is adjusted to have enough energy for ablation, for example, by estimating the energy hitting the target neural structure based on the relative location of the target neural structure. Optionally, the energy transmitter parameters (e.g., phase and/or amplitude and/or apodization (e.g., strength) of elements) are modified to reflect adjustment of the beam position based on the sensor data.

Optionally, at 308, blocks 304, 208 and/or 306 are repeated as part of the motion tracking and/or beam adjustment.

Alternatively, the method of FIG. 3 is performed without the catheter having position sensors (e.g., block 304). Optionally, the energy beam is guided to the target neural structure based on images. For example, the energy beam is guided to the celiac ganglion based on recognition of the location of the celiac ganglion using images (e.g., images of arteries surrounding the celiac ganglion). Image recognition may be performed manually (e.g., by the operator), automatically by software, or semi-automatically. For example, image recognition is performed based on ultrasound images containing one or more of: the aorta, the celiac ganglion (or other target ganglia), branches of the aorta, branches of the celiac artery, or other anatomical landmarks. The location of the target neural structure may be estimated based on the identified structural landmarks within the images. Beam adjustment may be performed during treatment, based on images acquired during treatment (e.g., in real time, using fluoroscopy) and/or based on images acquired before treatment (e.g., CT, MRI) with estimated compensation for motion.

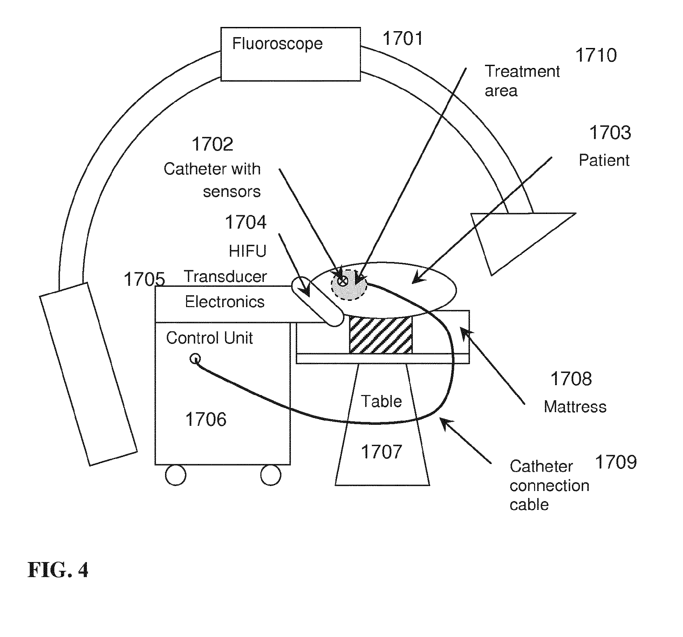

Reference is now made to FIG. 4, which is a schematic illustration of some systems for nerve ablation in a catheterization laboratory, according to some embodiments of the present invention. The system of FIG. 4 may be used, for example, to perform the treatment based on the method of FIG. 3.

Optionally, the system and/or method described with reference to FIG. 3 and/or 4 may form a virtual trap around the target neural structure, so that the system and/or method remains locked onto the target neural structure. Movement of the neural structure (e.g., due to respiration, heartbeats, gastric, voluntary motion, involuntary motion, or other movements) is tracked so that the energy beam remains targeted onto the neural structure during and/or after the motion. The sensors may be tracked, so that the energy path to the target structure is relative to the location of the sensors, instead of in absolute coordinates (which might not compensate for change).

The illustration shows the position of the fluoroscope as at 1701 used to align a catheter 1702 and an external HIFU transducer 1704 (for generating the ablation energy) with a target treatment location 1710 inside a patient 1703. The catheter with optional position sensor may be connected to a control unit 1706 using a connecting cable as at 1709. Optionally, a mattress has recesses 1708 to allow HIFU transducer 1704 to be positioned appropriately, for example, on the posterior and/or lateral side of the patient at an approximate 45 degree angle with the coronal plane, in other words rotated 45 degrees about the inferior superior axis as illustrated. The HIFU transducer may be powered by electronics 1705 which are in turn connected to the control unit 1706. The system may consist of four main components: catheter, transducer, control unit, and/or optional mattress.

The path of energy delivery may be planned automatically by software and/or manually by the operator. Optionally, the path of the delivered energy is displayed on a screen. Optionally, the planned path is displayed. Alternatively or additionally, ablated areas are displayed, for example, with different colors. Optionally, the path is correlated and/or registered with an anatomical image of the patient, for example, obtained using CT, MRI and/or fluoroscopy. The images may be taken before treatment and/or in real time during treatment.

Additional details of the system are described, for example, in Applicant's PCT Application PCT/IB2013/061457.

Additional details of the mattress are described, for example, in Applicant's PCT Application PCT/IB2013/061435.

Optionally, the externally applied HIFU system with internal catheter described with reference to the mentioned PCT Applications of the Applicant is adapted for targeting the ganglia, for example, the celiac, superior mesenteric, inferior mesenteric, and/or aorticorenal.

Optionally, the external applied HIFU system is aimed from the back of the patient and/or from the side of the patient, to avoid air filled intestines on the path of the ultrasound energy towards the ganglia. Alternatively, the intestines are filled with a suitable fluid (e.g., saline, water, other fluids) for transmission of the ultrasound energy, and the HIFU system is set up for transmission from the front of the patient, through the fluid filled intestines to the ganglia.

Optionally, the external applied HIFU system is programmed to have a safety region during the ablation. Optionally, the aorta and/or branch vessels are within the safety region. Alternatively, ultrasound energy transmission to the aorta and/or branch vessels is allowed and/or is part of the treatment scheme. Optionally, the aorta and/or branch vessels are monitored for damage. Optionally, energy delivery is programmed based on the fast blood flow and/or volume of flow of blood through the aorta and/or branch vessels. The flowing blood may dissipate accumulation of energy so that the aorta and/or branch vessels are not damaged.

Optionally, the aorta and/or branch vessels are monitored to measure the effect of treatment. Optionally, the catheter includes a sensor to measure effects on the aorta and/or blood vessel, for example, a temperature sensor. Optionally, the temperature within the aorta and/or branch vessels is monitored. The temperature may be monitored as a safety measure, to make sure that the blood within the aorta and/or branch vessels is not heated to the point of damage (e.g., coagulation, denaturation). The temperature may be indicative of the ablation effects at the ganglion.

Optionally, an energy transmitter is used to ablate the ganglion. Optionally, the focus size of the focus of the energy transmitted is predetermined to be large enough to ablate a required portion of the ganglion, for example, about 1mm radius, or about 2mm radius, or about 4mm radius, or about 7mm radius, or other smaller, intermediate, or larger sizes to perform the ablation. The selected radius may be more effective at a shorter duration, for example, by thermally affecting the treated ganglion at the same time.

Optionally, the path of the beam creates thermal spots, which may be combined to create the required geometry for ablation.

Optionally, the ablation is conducted in stripes (and/or other patterns), such that the stripes cross the required area of treatment at an effective speed. Optionally, the speed of ablation is determined by the power of transmission. Alternatively or additionally, the speed of ablation is determined by an estimate of the power of the beam at the target. Alternatively or additionally, the speed is determined by the number of elements transmitting to the target.

Optionally, the elements selected for transmission to the target are selected according to an estimated path from each element or group of elements to the target.

Optionally, the transmitter is positioned in a posterior-lateral position, such that the beam transmitted has less chances of hitting obstacles, such as the spine.

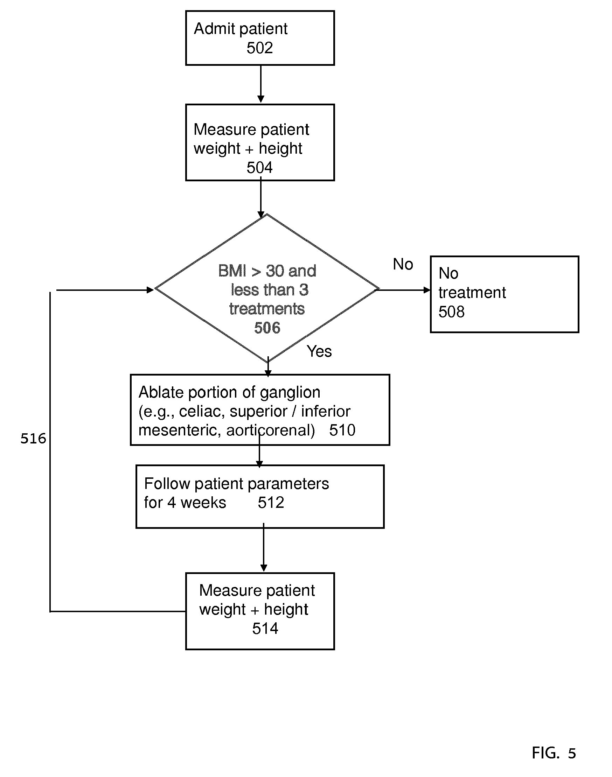

Reference is now made to FIG. 5, which is a method for treating an obese and/or overweight patient, in accordance with some embodiments of the present invention. The method of FIG. 5 may be based on the method of FIG. 2.

Optionally, at 502, the patient is admitted for possible treatment, for example, the patient suffered a catastrophic event secondary to obesity and ended up in the emergency room, the patient has been referred by a physician for evaluation of possible treatment, the patient failed other obesity treatments, the patient self chooses to conduct a weight loss procedure, and/or other factors.

Optionally, at 504, the patient is screened for treatment by ablation of nerve structures.

Optionally, the patient is screened for obesity.

Optionally, the weight and/or height of the patient are measured. Optionally, the BMI of the patient is calculated.

Optionally at 506, the patient is evaluated against obesity criteria and/or other procedure inclusion criteria, for example, based on BMI, absolute weight, or other factors. For example, BMI > 25, BMI > 30, BMI > 35, BMI > 40, or other criteria.

Optionally, at 508, the patient has not been found to meet the treatment criteria. Treatment is not performed.

Alternatively, at 510, the patient has been found to meet the treatment inclusion criteria.

The patient is treated by ablation of one or more nerve structures, for example, the celiac, super mesenteric, inferior mesenteric, and/or aorticorenal ganglia. For ganglia that are located bilaterally, one or both may be ablated. The ganglion may be ablated entirely or partially, for example, at least about 10%, or about 25%, or about 50%, or about 75%, or about 90%, or about 100%, or other smaller or intermediate values. Other structures may be ablated, for example, splanchnic nerves, renal nerves, and/or regions of the sympathetic chain.

The ablation may proceed, for example, based on systems and/or methods described herein, and/or other suitable methods and/or systems.

Optionally, at 512, the patient is followed up. Optionally, the patient is followed up after 4 weeks, 12 weeks, or other time periods.

Optionally, at 514, the patient is re-evaluated. Optionally, one or more parameters are re-measured.

Optionally, the height and/or weight are re-measured. Optionally, the BMI is re-calculated.

Optionally, one or more metabolic parameters are tracked, for example, measurements related to insulin resistance (e.g., OGTT, HbA1c), grehline level, lipin level, or other levels. The metabolic parameters may be used, for example, to track progress of the patient to reach a target metabolic parameter level, may be used as treatment inclusion criteria, and/or as treatment exclusion criteria.

Optionally, at 516, the patient is evaluated for another treatment. Optionally, the re-evaluation criteria are compared against the initial inclusion criteria (e.g., block 506). Optionally, the BMI of the patient is compared against the inclusion criteria. Alternatively, the re-evaluation criteria are compared against a target measurement.

Optionally, the patient is denied treatment if the number of treatments already performed has exceeded a predefined threshold, for example, above 1, 2, 3, 4, or other number of treatments. The patient may be denied treatment based on exceeding the number of treatments even if the patient meets other inclusion criteria.

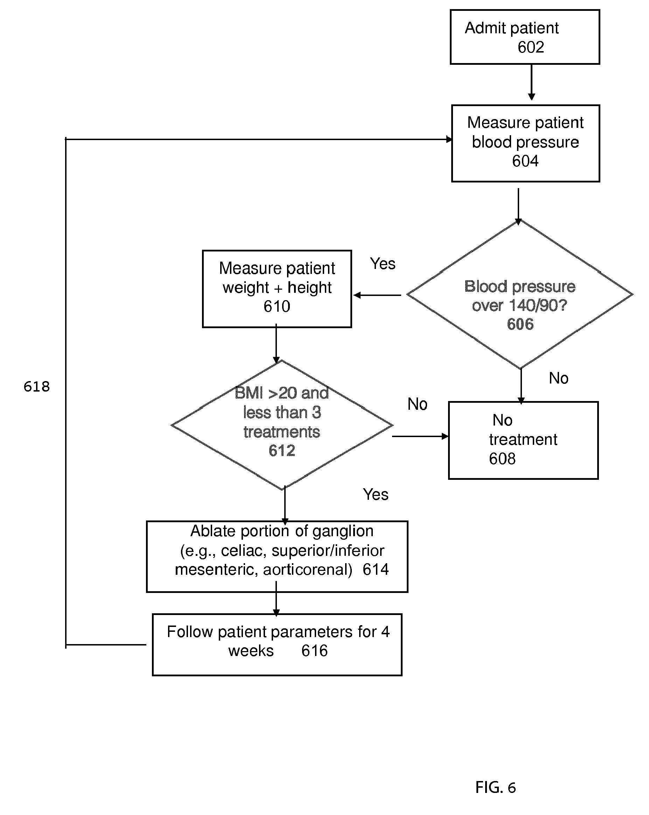

Reference is now made to FIG. 6, which is a method for treating a hypertensive patient, in accordance with some embodiments of the present invention. The method of FIG. 6 may be based on the method of FIG. 2.

Optionally, at 602, the patient is admitted for possible treatment, for example, the patient suffered a catastrophic event secondary to hypertension and ended up in the emergency room (e.g., stroke), the patient has been referred by a physician for evaluation for possible treatment, the patient failed other hypertensive treatments, or other factors.

Optionally, at 604, the patient is screened for treatment by ablation of nerve structures.

Optionally, the patient is screened for hypertension. Optionally, the patient is screen for drug resistant hypertension, for example, the patient is currently (or has been on) at least 3 different anti-hypertension drugs without acceptable reduction in blood pressure.

Optionally, the blood pressure of the patient is measured.

Optionally at 606, the patient is evaluated against a first set of hypertension criteria and/or other procedure inclusion criteria, for example, based on blood pressure, adverse events secondary to high blood pressure, or other factors. For example, blood pressure > 140/90, > 160/100, or other measurements.

Optionally, the patient is screened for having enough weight so that the patient may experience weight loss from the treatment without possibly harmful effects. Optionally, the patient is evaluated (e.g., measured) for a suitable BMI, for example, above a threshold such as > 18.5, > 20, > 22, > 24, > 25, or other BMI values. Alternatively or additionally, the patient is evaluated for having enough weight such that a reduction of about 5 (kilograms) Kg, or about 7Kg, or about 10Kg, or about 15Kg, or other weight loss values maintain the patient above the underweight category. Alternatively or additionally, the patient is evaluated for being able to lose about 2%, or about 5%, or about 10%, or about 15%, or about 30%, or about 45%, or any other values of weight loss, without becoming underweight.

The patient may be obese, in which case weight loss may be a desirable treatment effect. Optionally, at 608, the patient has not been found to meet the first set of treatment criteria. Optionally, treatment is not performed.

Alternatively, at 610, the patient has been found to meet the first set of hypertension treatment inclusion criteria.

Optionally, at 612, the patient is evaluated for a second set of hypertension treatment inclusion criteria. Optionally, the second set of criteria may verify that the patient is able to withstand secondary treatment effects, either desirable or side effects. Optionally, the patient is screened for enough weight so that the patient may experience weight loss from the treatment without harmful effects. For example, the patient is evaluated for a BMI > 18.5, > 20, > 22, > 24, > 25, or other BMI values. The patient may be obese, in which case weight loss may be a desirable treatment effect.

Alternatively, if the patient has not been found to satisfy the second set of treatment inclusion criteria, the patient is denied treatment (block 608).

Optionally, at 614, the patient is treated by ablation of one or more nerve structures, for example, the celiac, super mesenteric, inferior mesenteric, and/or aorticorenal ganglia. For ganglia that are located bilaterally, one or both may be ablated. The ganglion may be ablated entirely or partially, for example, at least about 10%, or about 25%, or about 50%, or about 75%, or about 90%, or about 100%, or other smaller or intermediate values. Other structures may be ablated, for example, splanchnic nerves and/or renal denervation and/or regions of the sympathetic chain.

The ablation may proceed, for example, based on systems and/or methods described herein, and/or other suitable methods and/or systems.

Optionally, at 616, the patient is followed up. Optionally, the patient is followed up after 4 weeks, 12 weeks, or other time periods.

Optionally, at 618, the patient is re-evaluated. Optionally, one or more parameters are re-measured.

Optionally, the blood pressure is re-measured (block 604).

Optionally, one or more metabolic parameters are tracked, for example, measurements related to insulin resistance (e.g., OGTT, HbA1c), grehline level, lipin level, or other levels. The metabolic parameters may be used, for example, to track progress of the patient to reach a target metabolic parameter level, may be used as treatment inclusion criteria, and/or as treatment exclusion criteria.

Optionally, the patient is evaluated for another treatment session. Optionally, the re-evaluation criteria are compared against the initial inclusion criteria (e.g., block 606). Optionally, the blood pressure of the patient is compared against the inclusion criteria. Alternatively, the re-evaluation criteria are compared against a target measurement.

Optionally, the patient is denied treatment when the number of treatments already performed has exceeded a predefined threshold, for example, above 1, 2, 3, 4, or other number of treatments. The patient may be denied treatment based on exceeding the number of treatments even if the patient meets other inclusion criteria.

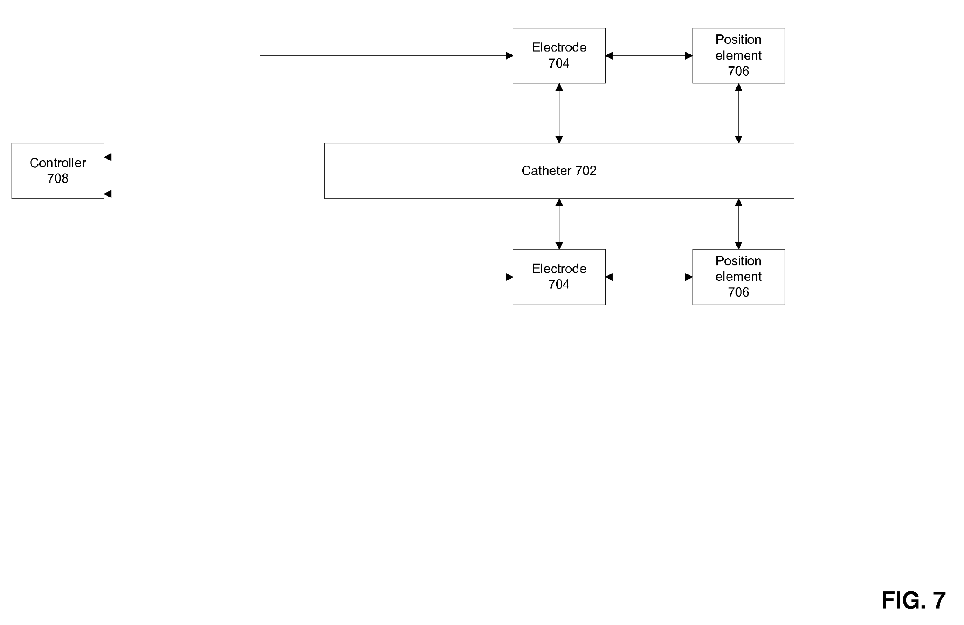

Reference is now made to FIG. 7, which is a schematic block diagram of a system 700 for ablation of nerve structures form within the aorta and/or aorta branch vessels, in accordance with some embodiments of the present invention.

System 700 comprises a catheter shaft 702 sized and/or formed for insertion into the target vessels such as the aorta and/or aorta branch vessels. Different catheters 702 may be designed for different vessels, or the same catheter may be used for several or all vessels. Catheter 702 is designed for insertion into: the aorta, the celiac trunk (or branches thereof: the common hepatic artery, the left gastric artery, the splenic artery), the superior mesenteric artery, the renal arteries, and/or the inferior mesenteric artery, and/or branches thereof. Catheter 702 may be designed to fit based on size, and/or may be specially formed for easy insertion into the vessel based on the vessel anatomy shape.

One or more electrodes 704 are positioned optionally at the distal end portion of catheter 702. Alternatively or additionally, a ground electrode may be positioned outside the body. The arrangement may depend on the application of energy, using a biopolar and/or unipolar RF energy and/or another frequency of electrical energy, and/or direct current (DC) delivery scheme. Optionally, electrodes 704 are positioned and/or arranged to form an axis between any two of multiple electrodes 704 when distal end portion of catheter 702 is positioned inside the target vessel, and/or otherwise geometrically encompass the target nerve structure. Optionally, one or more electrodes 704 used for energy delivery are designed to be positioned within the aorta, and another one or more electrodes are designed to be positioned within one or more aorta branch vessels. Alternatively, all electrodes 704 used for energy delivery are designed to be positioned within the aorta. Alternatively, all electrodes 704 used for energy delivery are designed to be positioned within the aorta branch vessels.

Optionally, electrodes 704 are arranged in a spaced apart arrangement along the distal end portion of catheter 702. The spacing may be selected to position a first set of electrodes within the aorta when a second set of electrodes is positioned within the aorta branch vessel. Other vessel arrangement may also be possible, for example, within the celiac artery and within branches of the celiac artery, or within two different aorta branch (optionally not within the aorta), or within two different branches of the celiac artery, or other arrangements. The spaced apart arrangement may be selected to encompass the neural structure by forming an axis between the first set of positioned electrodes and the second set of positioned electrodes. The first set of electrodes may be positioned at one end of the axis, and the second set of electrodes is positioned at an opposite end of the axis. Several axes may be formed between different electrodes, with different electrodes on opposite ends of each axis.

Optionally, the curvature of electrodes 704 is designed to contact the inner wall of the target vessel, for example, the aorta, and/or aorta branch vessels, and/or celiac trunk branch vessels. The curvature to fit vessels of different diameter may improve contact of each electrode 704 with the vessel wall.

Optionally, the radial positions of the electrodes 704 along catheter 702 are selected for the electrodes to generally face the target neural structure, for example, in a straight line. Alternatively, electrodes 704 are positioned at various radial positions (e.g., to cover all target locations), for example, in a spiral pattern. The selection of specific electrodes 704 to target the neural structure may be selected after catheter 702 has been inserted into treatment position.

One or more optional positioning elements 706 are coupled to each electrode and/or set of electrodes 704 and/or catheter shaft 702 for securing the position of electrodes 704 within the target vessel, and/or for positioning electrodes 704 for contact with the inner wall of the target vessels. Optionally, positioning elements 706 pushe electrodes 704 against the vessel wall for good electrical coupling.

Optionally, positioning elements 706 are expandable cages, for example, self expanding made out of nickel-titanium. Positioning elements 706 may be located at the distal end region of catheter shaft 702 and/or at a more proximal region of catheter shaft 702. The different locations of positioning elements 706 may be designed for different vessels, based on expansion diameter, applied force, length and/or shape. For example, distal positioning element 706 for the celiac trunk, and proximal positioning element 706 for the aorta. The distal and proximal elements 706 may form an angle between the surfaces of the corresponding electrodes 704 in the different vessels. The electrodes 704 may surround the target nerve structure.

Other designs of positioning elements 706 include, for example, the shape of the shaft of the catheter (e.g., at the location of electrode coupling or nearby), a balloon, or other suitable designs.

A controller 708 is in electrical communication with electrodes 704, and programmed to apply electrical energy to one, two or more of electrodes 704 to ablate the neural structure. Optionally, controller 708 is an RF generator, or controller application of RF energy, and/or another frequency of electrical current, and/or direct current. Energy may be applied when electrodes 704 are arranged as polar and/or bipolar. Controller 708 may be programmed by software (or a memory and/or external media) and/or hardware.

Optionally, controller 708 activates one or more bipolar RF pulses from pairs of electrodes 704 so that the electrical current passes through the region between electrodes 704 and at least partially ablates the target nerve structure.

Optionally, controller 708 delivers energy to different electrodes 704. Optionally, energy is delivered to those electrodes 704 forming the geometrical encompassing arrangement and/or axis surrounding the neural structure.

Optionally, controller 708 applies the energy in an amount to ablate at least a portion of the neural structure. Optionally, the energy is applied without significantly ablating tissue surrounding the at least one neural structure. Optionally, energy is applied without damaging the aorta and/or aorta branch vessels.

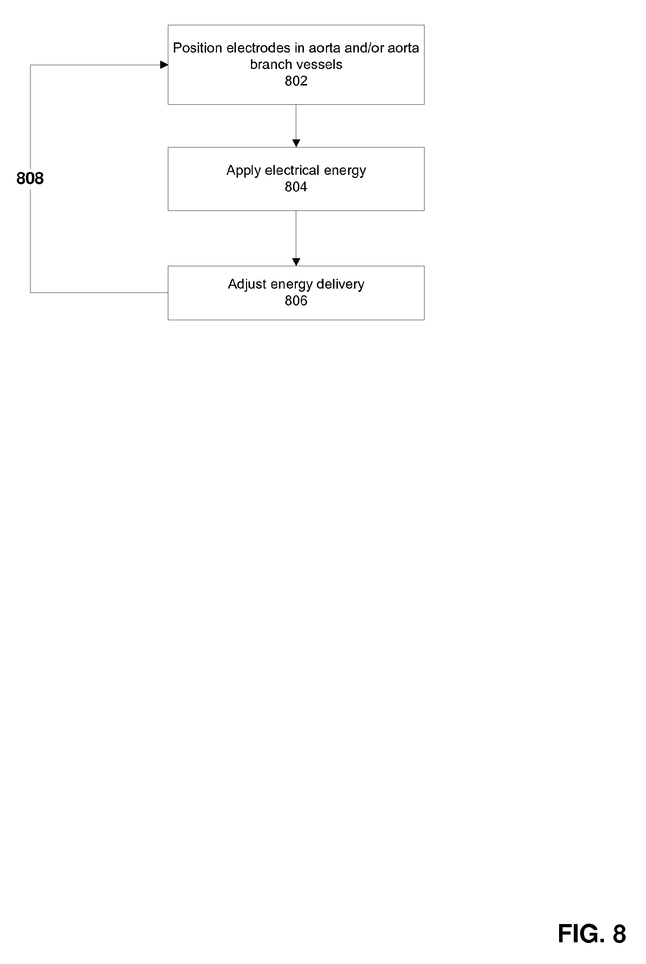

Reference is now made to FIG. 8, which is a flowchart of a method of ablating one or more neural structures by energy delivery through electrodes, in accordance with some embodiments of the present invention. The method of FIG. 8 may be performed, for example, by system 700 of FIG. 7, and/or by other devices as described herein, or other suitable devices. The method of ablation of FIG. 8 may be used for ablation, as described with reference to FIGs. 2, 5 and/or 6, and/or other suitable methods described herein.

The method of FIG. 8 and/or system 700 of FIG. 7 may be designed for ablation of neural structures in delicate areas. Optionally, the neural structures are one or more of: celiac ganglion, superior mesenteric ganglion, inferior mesenteric ganglion, and/or aorticorenal ganglion. Optionally, the delicate areas are the aorta and/or aorta branch vessels, and/or walls thereof.

At 802, electrodes are positioned within the aorta and/or aorta branch vessel. The electrodes are positioned to apply energy to ablate the target neural structure. Optionally, the electrodes are positioned in proximity of the neural structure. Optionally, the electrodes are positioned around the neural structure, to geometrically encompass the neural structure, and/or so that the neural structure lies along or in proximity to the axis between any two of the electrodes. For example, for two electrodes forming a straight line between them (i.e., axis), the target nerve structure may be less than about 10 mm away from the axis, or about 25 mm, or about 50 mm, or about 70 mm, or other smaller, intermediate or larger distances.

Optionally, the electrodes are positioned within the blood vessels, about 20 mm or more from the nerve structure, or about 10 mm or more, or about 30 mm or more, or about 50 mm or more, or other smaller, intermediate or larger distances.

Optionally, the electrodes are positioned for good contact with the inner wall of the blood vessels. For example, by deploying positioning elements 706 of FIG. 7.

Not necessarily limiting examples of vascular positioning of electrodes include:
Aorta and/or celiac artery and/or left gastric artery and/or common hepatic artery and/or splenic artery for ablation (part thereof or entirely) of one or both of the celiac ganglia.
Aorta and/or superior mesenteric artery for ablation (part thereof or entirely) of the superior mesenteric artery ganglion.
Aorta and/or interior mesenteric artery for ablation (part thereof or entirely) of the interior mesenteric artery ganglion.
Aorta and/or renal artery (left or right) for ablation (part thereof or entirely) of the corresponding aorticorenal ganglion.

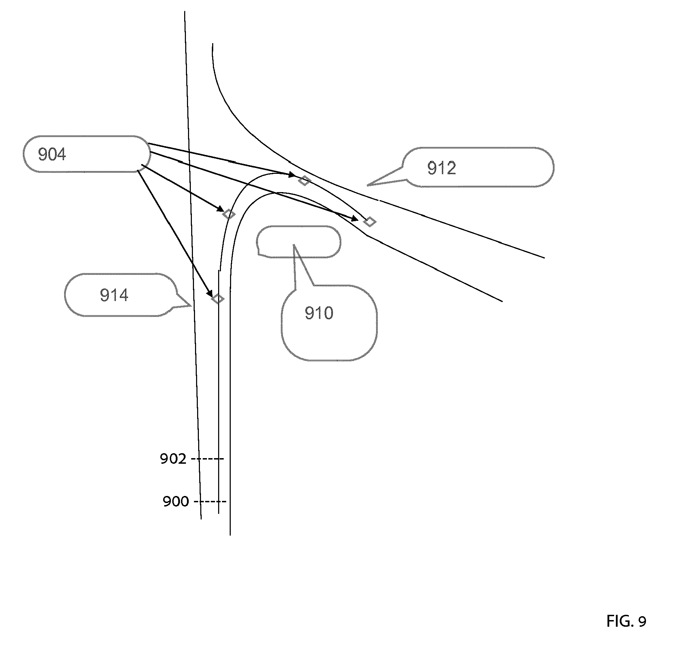

One or more electrodes may be positioned in the celiac artery, and one or more electrodes in the aorta (e.g., as shown in FIG. 9), so that the celiac ganglion is substantially between the electrodes, or near the axis drawn between the electrodes.

One or more electrodes may be positioned at one of the branches of the celiac trunk (left gastric artery, common hepatic artery, or splenic artery) and one or more electrodes in the celiac artery.

Other vessels may be used as sited for ablation. Different electrodes may be positioned in different vessels.

Positioning may be performed, for example, using real-time imaging modalities (e.g., fluoroscopy).

At 804, electrical energy is applied through the positioned electrodes. A bipolar and/or unipolar sequence may be applied. Optionally, the electrical energy is applied in an amount to ablate at least a portion of the target neural structure without significantly ablating tissue surrounding the at least one neural structure and/or without damaging the aorta and/or aorta branch vessels.

Optionally, current is applied between the electrodes (e.g., as shown in FIG. 9), so that one electrode is positive and another is negative, to drive current through the celiac ganglion (or other ganglions) to deactivate nerve cells. Current passing through the ganglion trapped between the electrodes is ablated. Alternatively, current is driven using unipolar electrodes, optionally alternating between the different electrodes (or all, or some at once). Optionally, energy is delivered at a non-ablating dose from the first electrode, and within a short period of time (and/or simultaneously) delivered at the non-ablating dose (same or another dose) from the second electrode, so that the region proximal to both electrodes is exposed to a longer duration of combined energy. The combined energy region may be ablated by the combined energy dose which is selected to be effective for ablation of the target nerve structure. Areas affected by energy delivery from only one of the electrodes may not be damaged.

Optionally, the unipolar pulse sequence from each electrode (using an externally applied electrode as ground) is applied so that the energy is delivered at a setting (e.g., power, duration) where each individual pulse is unable to cause significant tissue damage (e.g., at the vessel wall), but regions receiving multiple pulses are ablated by the total energy of the multiple pulses, for example, at the ganglion. In this manner, regions covered by more than one electrode may be ablated.

Optionally, current is applied (e.g., duration, amplitude) which does not cause damage to tissues when delivered by a single electrode, but ablates the ganglion (or portions thereof) when combined with other electrodes.

Optionally, the catheter contains multiple electrodes, and/or multiple electrodes are placed in position against the inner vessel wall. Optionally, two or more of the electrodes are selected (e.g., automatically by controller 708, manually by the user, and/or other methods) for energy delivery. Energy may be delivered to the selected electrodes.

Electrodes may be selected, for example, based on the quality of the contact with the inner wall, based on the position relative to the nerve structure, based on the part of the neural structure for ablation, selected together to surround the neural structure, or other selection factors. Optionally, two or more electrodes forming the axis are selected.

Optionally at 806, energy delivery is adjusted. Optionally, energy delivery is stopped, for example, if the target nerve structure has been ablated. Alternatively energy delivery is changed, for example, different parameters of energy delivery are selected, and/or different electrodes are selected. Energy delivery may be dynamically adjusted, for example, alternating between different electrodes encompassing the nerve structure so that the nerve structure is ablated without energy delivery to surrounding tissues to ablate the surrounding tissues.

Optionally, electrical energy is dynamically adjusted (e.g., duration, amplitude, frequency, power level, duty cycle, and/or other parameters) to alternate application of the electrical energy to different electrodes. Optionally, the adjusting is performed to selectively damage the neural structure over surrounding tissues. The variation in energy delivery electrodes may prevent or reduce energy accumulation at a single region. Energy levels may not reach a level high enough to damage non-nerve tissue (e.g., blood vessel wall). Energy application without the adjusting (e.g., to a single electrode, to two electrodes or more) may damage the blood vessel and/or surrounding structure.

Optionally, at 808, one or more of 802, 804 and/or 806 are repeated. For example, electrodes are repositioned (block 802) and/or electrically energy is reapplied (block 804) as part of the energy delivery adjustment (block 806).

Reference is now made to FIGs. 9-14, which are different catheter designs for ablation of nerve structures. The catheters may be used with controller 708 of FIG. 7. The catheters may be used to perform the ablation method of FIG. 8, and/or the treatment of FIGs. 2, 5 and/or 6.

Reference is now made to FIG. 9, which is a schematic illustration of a catheter 900 designed for ablation of nerve structures, in accordance with some embodiments of the present invention. Catheter 900 has multiple spaced part electrodes 904 for energy delivery, along a shaft 902.

Catheter 900 may be used, for example, to better encompass the target nerve structure. Multiple electrodes may encompass the target nerve structure. One, two, a subset, or all of the spaced apart electrodes 904 may be activated to delivery RF energy to ablate the target nerve structure. The electrodes applying the energy may be dynamically selected, so that the nerve structure is ablated without ablation of nearby tissues.

In one example, as shown, catheter 900 is positioned so that electrodes 904 encompass a celiac ganglion 910. Electrodes 904 may be positioned within a celiac artery 912 and/or aorta 914. For example, electrodes 904 are positioned along aorta 914, around a bend and within celiac artery 912 to encompass celiac ganglion 910.

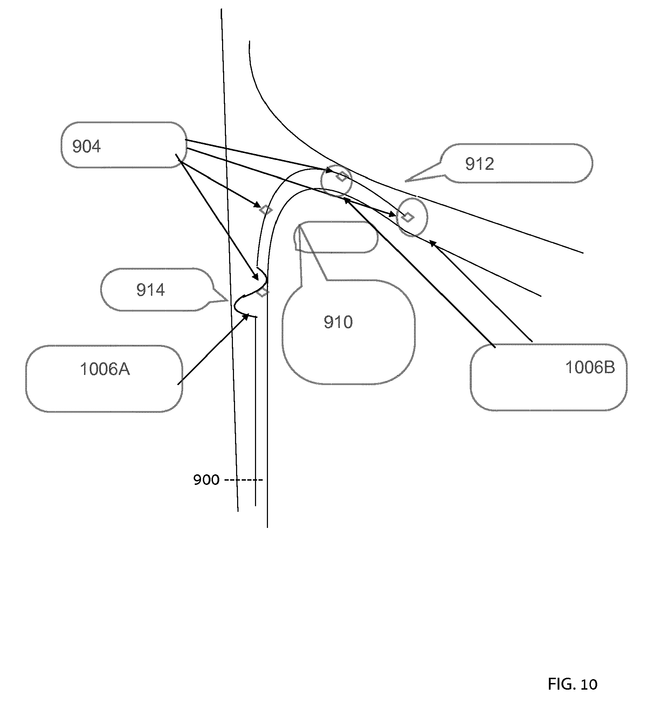

Reference is now made to FIG. 10, which is an illustration of catheter 900 of FIG. 9, with one or more positioning elements 1006A-B (e.g., as described with reference to positioning elements 706 of FIG. 7), in accordance with some embodiments of the present invention.

Optionally, positioning elements are designed for securing the position of catheter 900 in the blood vessel. Alternatively or additionally, positioning elements are designed for positioning the electrode against the inner vessel wall.

For example, positioning element 1006A is designed for positioning within aorta 914. Optionally, positioning element 1006A is designed to allow blood to continue flowing through aorta 914. Optionally, positioning element 1006A is designed to push one or more electrodes 904 against the inner aortic wall towards celiac ganglion 910. For example, positioning element 1006A is a helical contract structure, for example, made out of nickel-titanium. The helical contact structure may expand to the size of aorta 914 to apply a force against the inner aorta wall.

In another example, positioning element 1006B may be designed for positioning within celiac artery 912. Blood flow may be temporary halted within celiac artery 912. Positioning element 1006B may be an expandable balloon. Electrodes 904 may be positioned on the balloon itself, or the expanding balloon may force electrodes 904 towards the vessel wall.

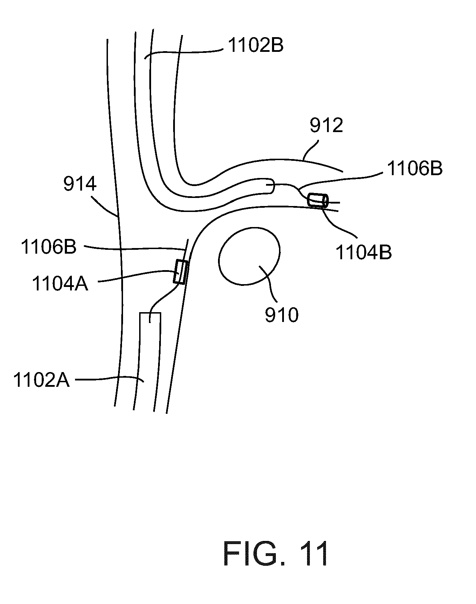

Reference is now made to FIG. 11, which is an illustration of two catheters 1102A-B for ablation of nerve structures, in accordance with some embodiments of the present invention.

Each catheter 1102A-B may have at least one electrode 1104A-B. The respective electrode 1104A-B may be coupled to a shaft 1106A-B. Catheters 1102A-B may be designed to position deployed electrodes 1104A-B against respective inner vessel walls. Each catheter 1102A-B may be maneuvered independently.

Optionally, each catheter 1102A-B is designed for insertion into a different vessel. For example, catheter 1102A is designed for deployment in aorta 914, for example, by access through the femoral artery. For example, catheter 1102B is designed for deployment in celiac artery 912, for example, by access through the radial artery. Catheters 1102A-B may have different sizes, and/or shapes to help in navigation and/or deployment to the target vessel. Electrodes 1106A-B are positioned to form an axis and/or encompass the target nerve structure, such as celiac ganglion 910. For example, to ablate celiac ganglion 910, catheters 1102A-B may be positioned so that celiac ganglion 910 (or portions thereof) are in between electrodes 1104A-B. For example, one catheter 1102 may be positioned at the common hepatic artery, and another at the splenic artery. In another example, one catheter 1102 may be positioned at the celiac artery, and another one at the splenic artery.

Optionally, energy application to electrodes 1106A-B on different catheters 1102A-B is controlled by a single controller, for example, controller 708 of FIG. 7. Energy delivery may proceed, for example, as if both electrodes were part of a single catheter. Energy applied from electrodes 1106A-B may ablate celiac ganglion 910 without damaging the aorta and/or other major blood vessels.

It is noted that more than 2 catheters may be used. For example, a third (or more) catheter may be positioned in the superior mesenteric artery, within the aorta the celiac branch, within a branch of the celiac artery, or other locations.

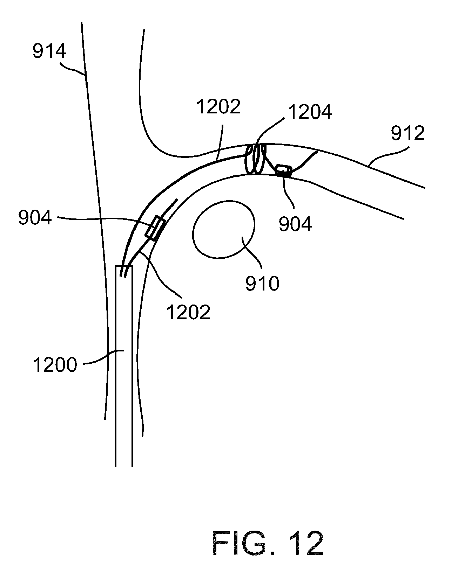

Reference is now made to FIG. 12, which is yet another schematic illustration of a catheter 1200 design for ablation of nerve structures, in accordance with some embodiments of the present invention. Catheter 1200 has multiple extensions 1202, for example, two, three, four or more. Each extension 1202 may be independently deployed, all extensions may be deployed together, one extension may be deployed leaving the rest behind, or other arrangements. The lengths of extensions 1202 may be varied during deployment.

Optionally, extensions 1202 are designed for insertion into and/or positioning inside different vessels, for example, one extension 1202 for deployment within aorta 914 (e.g., short length extension), and another extension for deployment within celiac trunk 912 (e.g., longer length extension). Extensions 1202 may be formed into predefined curvatures and/or shapes, so that upon deployment, respective extensions 1202 are more easily maneuvered into respective vessels.

In one example, the distal end region of catheter 1200 is maneuvered into celiac trunk 912. One extension 1202 with electrode 904 is positioned into one branch (e.g., left gastric artery). A different extension 1202 with a different electrode 904 is positioned in another branch (e.g., common hepatic artery). Optionally, a third extension with a third electrode is positioned in yet another branch (e.g., common hepatic artery). Activating the bipolar RF current between electrodes 904 may drive current through the target region between the electrodes, crossing celiac ganglion 910 in one or many paths, and ablating celiac ganglion 910.

Optionally, electrodes 904 are located on the distal end of extensions 1202, for example, one or more electrodes 904 per extension 1202. Energy may be applied to electrodes 904 to ablate the target neural structure (e.g., celiac ganglion 910) as described herein, using bipolar RF energy, unipolar RF energy, other frequency electrical energy (unipolar or bipolar), and/or DC (direct current) electrical energy.

Optionally, extensions 1202 have one or more positioning elements 1204, for example, a helical spring, as described herein. Helical spring 1204 may be sized and/or arranged to position and/or urge electrode 904 against the inner wall of celiac artery 912 towards celiac ganglion 910.

Catheter 1200 may provide greater flexibility and/or options to the operator in positioning electrodes 904 within different vessels and/or within the same vessel for ablation of the neural structure.

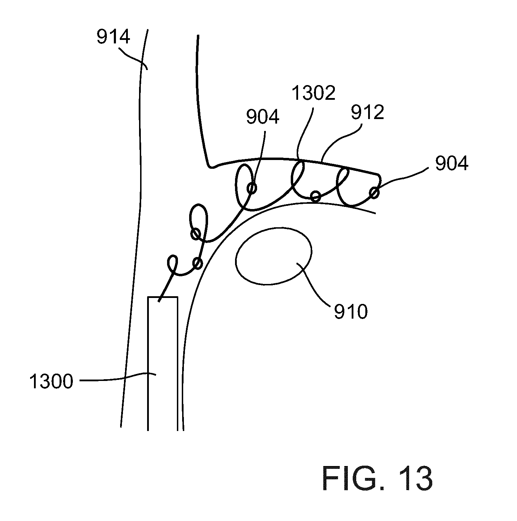

Reference is now made to FIG. 13, which is yet another schematic illustration of a catheter 1300 designed for ablation of nerve structures, in accordance with some embodiments of the present invention.

Optionally, electrodes 904 are arranged along one or more extensions 1302 so that electrodes 904 are positioned along the inner vessel wall at different locations along the circumference and/or along the length of the vessel. Optionally, extension 1302 is arranged to incorporate the function of the positioning element to apply a force to push electrodes 904 into contact with the inner vessel wall.

Extension 1302 may be, for example, a spring, a plurality of wires joined at one or both ends, an expandable cage, or other suitable structures.

Extension 1302 may be easier to deploy and/or position for ablation of the target neural structure. For example, extension 1302 may be released within the target vessel(s) in a single motion. For example, as shown, extension 1302 has been deployed within aorta 914, around a bend, and within celiac artery 912. Extensions 1302 may make it easier to ablate the target nerve structure by selection of a suitable subset of electrodes from the multiple available electrodes 904 at different positions within the vessel.

Optionally, the controller selects the appropriate electrodes 904 from which to apply energy to ablate the target neural structure, such as celiac ganglion 910. Optionally selection is determined manually. Alternatively or additionally, selection is determined automatically and/or semi-automatically, by analyzing parameters such as electrical coupling parameters (such as impedance) and/or other parameters such as geometry.

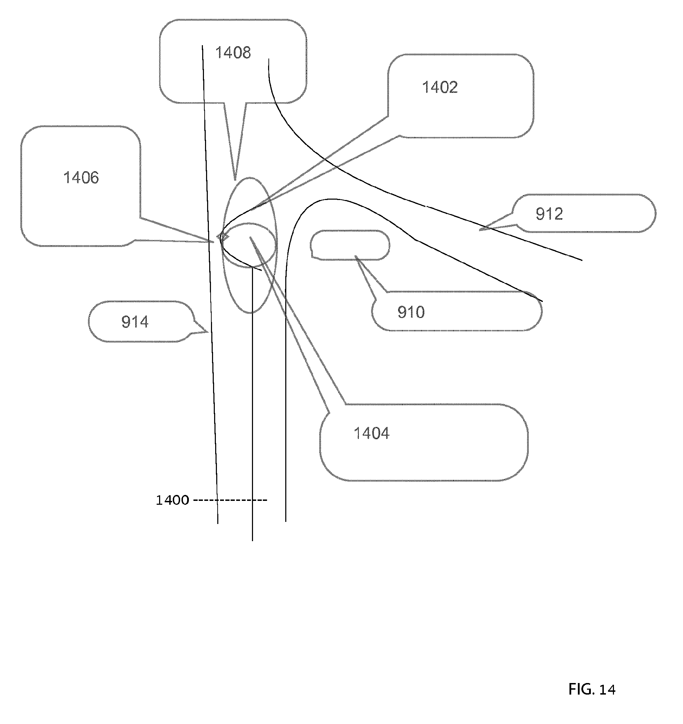

Reference is now made to FIG. 14, which is yet another schematic illustration of a catheter 1400 designed for ablation of nerve structures, in accordance with some embodiments of the present invention. Optionally, catheter 1400 is used with the ablation method described with reference to FIGs. 2, 3, 5, 6 and/or 8. The method of FIG. 8 may be used by interchanging the electrodes and/or RF energy with an emitter of the energy ablation modality of catheter 1400. Optionally, catheter 1400 is designed for ablation of specific ganglia from within predefined locations of the body. For example, the celiac, superior mesenteric, inferior mesenteric and/or aorticorenal ganglia, from predefined locations within the aorta and/or aorta branch vessels. Alternatively, catheter 1400 may be positioned within the esophagus.

Catheter 1400 comprises an ablation element such as transmitter 1402 arranged for intravascular deliver of focused ultrasound, phased array ultrasound, microwave, other electromagnetic energy, or other energy modality for ablation. Transmitter 1402 may be, for example, a phase array transmitter, a single element transmitter, an annular array transmitter, and/or a reflector. Optionally, transmitter 1402 may direct energy at a focal region containing the target nerve structure (e.g., celiac ganglion 910) located at least 3 mm, 5 mm, 7 mm, 10 mm, 20 mm, 50 mm, or other distances away from the inner surface of the blood vessel (e.g., aorta 914 and/or celiac artery 912). Alternatively or additionally, the focal region is between about 5 mm and 50 mm, or about 10 mm and 20mm, or other distance ranges from transmitter 1402. Optionally, the energy focus position may be adjusted during the treatment, for example, from about 5 mm to about 50 mm, or about 10 mm to about 20 mm, or other smaller, intermediate or larger ranges.

Optionally, catheter 1400 contains a positioning element (e.g., balloon 1408) with at least one lumen (e.g., hole 1404) to allow blood to flow through the lumen when expanded inside the blood vessel (e.g., aorta 914 and/or celiac artery 912). Optionally balloon 1408 has an expanded size and/or shape selected for securing the position of transmitter 1402 inside aorta 914 (or other vessel) when expanded. The balloon may be designed and/or made out of a material so that ultrasound energy is not absorbed and/or so that the balloon is not heated. Balloon 1408 may be the positioning element described herein.

Optionally, transmitter 1402 is located within the positioning element. Optionally, expanding the positioning element arranges transmitter 1402 into the proper shape for transmission of energy. For example, transmitter 1402 has a concave shaped focusing element that forms into the proper shape for transmitting focused energy.

Optionally, the positioning element is filled with a fluid suitable for transmission of the directed energy to the inner wall of the vessel, for example, the fluid may be saline, water, blood, or other fluids. Optionally, balloon 1408 is expanded by injection of the energy transmitting fluid.

Optionally, the distance of the focal region of transmitter 1402 is adjusted to different distances to ablate different parts of the neural structure. Alternatively or additionally, the applied energy is adjusted to ablate the neural structure. The size of the focal region may be, for example, between about 1 mm and 3 mm, or about 1 mm and 10 mm, or about 3 mm and 20 mm, or about 5 mm and 50 mm, or about 10 mm and 30 mm, or other ranges. Adjustment may be performed, for example, when the neural structure is too large for ablation with the applied energy beam. Optionally the energy beam is steered to direct the applied energy for ablation of the neural structure. Steering may be continuous and/or in segments, for example, sweeping across the length of the nerve structure and/or in steps (ablating one part, then moving to ablate another part).

Adjustment of the transmitter may be performed with a smaller focus area. Moving the focal area in and out of the tissue and/or up and/or down may cover the target treatment area within the ganglion. Optionally, the transducer is not moved and/or remains stationary, with reflectors of ultrasound on the catheter being moved, to allow directing the energy at other portions of the required target. Optionally, the reflectors are located in other vessels than the vessel containing the transducer, for example a transducer in the aorta and a reflector in the celiac trunk. Optionally the transducer elements and the reflectors are both manipulated to allow a combined effect of moving the focus of energy to a selected spot, and/or through a selected path.

Optionally, a radio-opaque marker 1406 on catheter 1400 may be used for aiming transmitter 1402 towards the ganglion. For example, under fluoroscopic imaging. Marker 1406 may be designed so that the direction of energy transmission is visible on the imaging device. Positioning may be manually performed by an operator based on the imaging, and/or automatically using other methods. Optionally, catheter 1400 has a mechanism to enable rotation of transmitter 1402 to aim the energy beam at the target ganglion. The angle of rotation may be indicated by marker 1406.

Optionally, a controller in electrical communication with catheter 1400 is programmed to adjust the focus, power of transmitter 1402, and/or other parameters. Optionally, the controller is programmed to move the focus of the energy to cover the target volume within the ganglion. The adjustment may be performed automatically, manually and/or semi-automatically. Optionally, the position and/or path of ablation are displayed on a monitor. Optionally, the monitor depicts the area which was exposed to the ablation energy. Optionally, the region of applied ablated energy is correlated and/or registered with an anatomical image of the patient's anatomy, for example, a real time fluoroscopic image, or a pre-recorded CT image.

Alternatively or additionally, catheter 1400 is designed to be positioned in lumens other than the aorta, for example, an aorta branch vessels, the esophagus, or other lumens. Optionally, transmitter 1402 is an ultrasound transmitter. When located in vessels other than aorta, hole 1404 may not be necessary, as temporary blockage of blood flow through the lumen may be tolerated. Optionally, transmitter 1402 is arranged to transmit the ultrasound beam to specific locations around the vessel (e.g., celiac trunk 912) to ablate the target nerve structure (e.g., celiac ganglion 910). Optionally, transmitter 1402 is arranged to transmit the ultrasound beam to two sides, or with a focus. Optionally, the focus is adjusted, for example, the energy level at the focus is adjusted, the frequency of ultrasound is adjusted, and/or the position of the focus is adjusted. The energy may be transmitted through a part of the circumference of the vessel, for example, an arc length of no more than about 15 degrees, or about 30 degrees, or about 45 degrees, or about 60 degrees, or other lengths. Optionally, the focus is adjusted to maneuver the treatment point into the target neural structure, for example, the celiac ganglion. Adjustment may be performed manually by the operator, automatically by a software module, or semi-automatically with the operator guiding the software module.

Additional embodiments, devices and/or features are now described with reference to FIGs. 2, 5, 6 and/or 8. A suitable catheter for performing the methods may be deployed through the arterial system, the venous system, and/or other tissues and/or other lumens (e.g., esophagus, intestines) to ablate portions or all of the sympathetic system ganglia. The catheter may be inserted, for example, through the femoral artery or radial artery to reach the aorta and/or branch vessels. Once in position within the aorta (e.g., in front of the target neural tissue), a cage may optionally be expanded within the aorta to maintain the position. The ablation source (e.g., ultrasound transmitter, x-ray transmitter, laser, or other suitable energy modalities) is positioned within the aorta, optionally opposite the target.

Optionally, the target distance of the transmitter is adjustable, for example, the ultrasonic transmitter may contain a deflectable ultrasonic lens and/or deflectable reflector. The focal distance of the beam may be modifiable, to ablate portions of the ganglion near the aorta and far from the aorta without increasing the applied energy.

Alternatively or additionally, the power is adjustable to change the ablation effect at the focal point. For example, the ultrasound transmitting catheter may be able to transmit at multiple power settings. The first power setting may ablate a portion of the celiac (or superior-mesenteric ganglion), and an additional power setting may ablate yet additional portion of the same. Together, the power settings may create a greater thermal effect to ablate the ganglion.

Optionally, each device has a predetermined focal depth, or a range of depths. Optionally, the devices are sold as a kit, for example, for treatment of one or more different ganglia. For example, one catheter type may be inserted for treatment of a first ganglia (and/or first part of the ganglion), and another catheter may be used to treat another ganglia (and/or another part of the same ganglion). The different catheters may be used during the same treatment session (e.g., to create a more thorough effect), or during different treatment session (e.g., if monitoring has determined that another session is required).

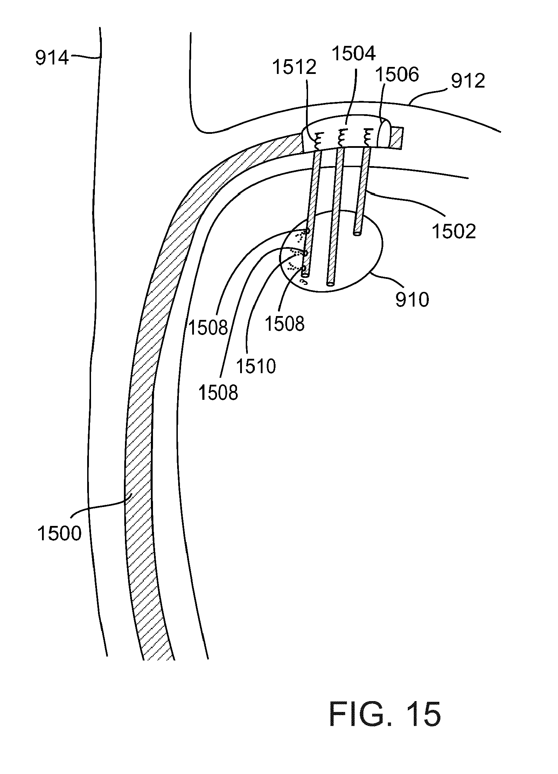

Reference is now made to FIG. 15, which is yet another suitable catheter 1500 for performing the methods described with reference to FIGs. 2, 3, 5, 6 and/or 8. Catheter 1500 has one or more micro-needles 1502. Optionally, micro-needles 1502 are encapsulated within a structure 1504 arranged towards a predefined direction with respect to the shaft of catheter 1500 and/or needles 1502. Structure 1504 may or may not be symmetrical. Optionally, needles 1502 are arranged to pierce through a portion of the circumference of the blood vessel, for example, an arc length of no more than about 15 degrees, or about 30 degrees, or about 45 degrees, or about 60 degrees, or other values.

Optionally, the diameter of needles 1502 is small enough so that after piercing through a blood vessel, the remaining hole is closed, plugged and/or clotted by the body before a significant amount of blood escapes. Use of the microneedles may not require repair of the blood vessel due to blood leaking through the holes caused by the needles.

Needle containing structure 1504 may be delivered into a target vessel by transvascular catheter 1500, for example, structure 1504 is located at a distal end of catheter 1500. Once the general location has been reached, structure 1504 may be oriented towards the target nerve structure. For example, as shown catheter 1500 is used to ablate celiac ganglion 910 from structure 1504 located within celiac artery 912. Catheter 1500 and/or structure 1504 may contain a radio-opaque marker 1506 to assist with orientation and positioning of the needle releasing portion of structure 1504. Micro-needles 1502 released from structure 1504 (e.g., by retraction of them micro-needles) pierce tissues to inject the target nerve structure, such as celiac ganglion 910.

Optionally, structure 1504 contains multiple sets of micro-needles 1502. Each set of needles 1502 may be deployed to different lengths, for example, to about 1 mm, or about 3 mm, or about 5 mm or about 7 mm, or about 10 mm, or about 15 mm, or about 20 mm, or about 30 mm, or other smaller, intermediate or larger distances. Or for example, deployed to about 3 mm to about 20 mm, or about 5 mm to about 15 mm, or other ranges. Optionally, length of needles 1504 is selected to be long enough to reach all portion of the target neural structure when structure 1504 is located within the vessel or lumen. Optionally, the different sets of needles 1502 are independently controlled or released, and/or controlled or released together. Alternatively or additionally, each needle 1502 (or at least some needles 1502) contains multiple holes 1508 along the shaft for releasing ablation drugs and/or chemical substances 1510 from the multiple exit points along the needle axis.

The needle containing structure 1504 (or catheter 1500) may be used for ablation of nerve structures as described herein. Optionally, the distal end portion of the catheter containing the needle structure is positioned in a vessel, for example, aorta 914, celiac artery 912 (or branches thereof), superior mesenteric artery, inferior mesenteric artery, renal artery, and/or other nearby lumens, such as veins, the esophagus, the stomach, or other lumens. Optionally, structure 1504 is positioned

(e.g., assisted by the radio-opaque marker 1506) so that micro-needles 1504 are deployed into the target tissue. Chemicals 1510 injected into the target tissue ablate the target neural structure.

Needles 1502 may be released (e.g., by the operator), for example, by an extraction mechanism 1512, for example, a spring, a remote controller that pushes or pulls the needle with precise motion, or other mechanisms. Optionally, needles 1502 are extracted in stages. For example, a first set of needles to verify the location of the needles within the target tissue. A nerve deactivating chemical (e.g., alcohol) may be injected into the target tissue to ablate the target nerve structure. Additional sets of needles may be extracted, for example, to inject the target tissue in several steps, such as to release chemicals deeper into the tissue. Alternatively, the same set of needles is expanded in multiple steps (or in a continuous manner) to inject the ablation chemicals into multiple locations at different distances from the catheter. In this manner, larger nerve structures may be partially or completely ablated. Optionally, needles 1502 (and/or extraction mechanism 1512) are arranged to be released to a predefined depth, for example, to a closer or deeper portion of the target neural structure. Alternatively, needles 1502 (and/or extraction mechanism 1512) may be deployed to varying depths until a maximum depth.

Optionally, the ablation drug is itself, or mixed with, a radio-opaque material or colored with a contrast agent. The injection and/or location of the injected chemicals may be visualized in the tissue, for example, by fluoroscopy. The injection and/or treatment may be controlled by the visual feedback.

Alternatively or additionally, needles 1502 are cryo-needles, for freezing surrounding. The cryo-needles may also inject chemicals in addition to the freezing.

Alternatively or additionally, needles 1502 contain electrodes for RF ablation.

Alternatively or additionally, needles 1502 are designed to heat surrounding tissue.

Alternatively or additionally, needles 1502 contain ultrasound attenuators. Needles 1502 may be wires, and may not need to contain a pass-through lumen.

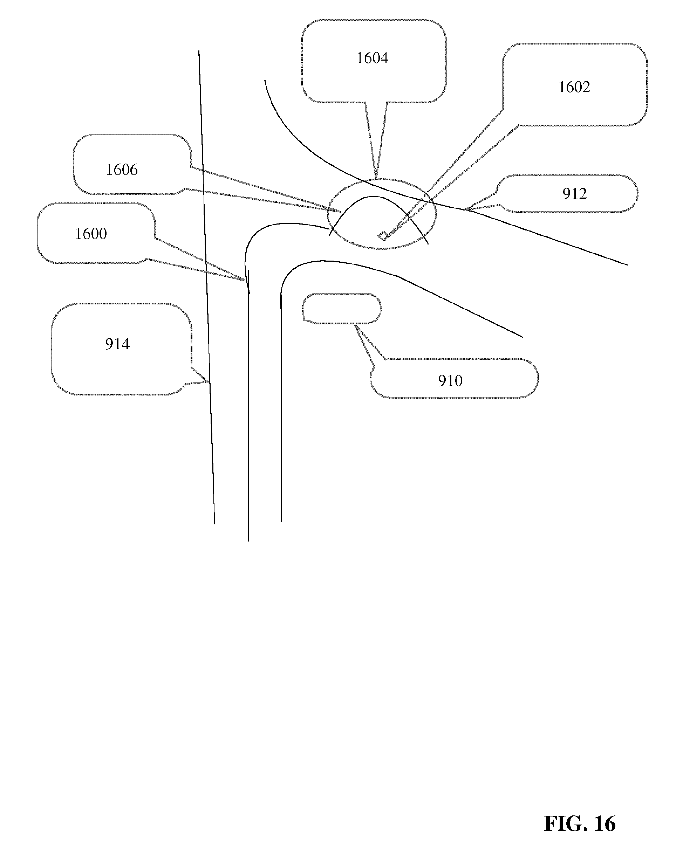

Reference is now made to FIG. 16, which is yet another suitable catheter 1600 for performing the methods described with reference to FIGs. 2, 3, 5, 6 and/or 8. Catheter 1600 may be positioned in small diameter vessels to ablate neural structures located far from the small diameter vessels. To ablate the far neural structure with a large distance focus, for example, about 5 mm, or about 10 mm, or about 20 mm, about 30 mm, about 50 mm, about 70 mm, or other distances, the transmitter used to form the focus may need to have a similar diameter as the distance to the focus. When the focus is large, the large transmitter may not be able to fit inside the vessel, or vessels that are large enough may not be suitable locations (e.g., aorta, esophagus).

Optionally, a transmitter 1602 of catheter 1600 is designed to address conflicting requirements. Optionally, transmitter 1602 is designed to have a small diameter (for example, insertion into vessels such as femoral artery and target vessels (e.g., celiac artery 912) without causing trauma), and transmitter 1602 is designed to have a large enough size to be able to focus energy deep into the tissue (e.g., without energy loss to the sides), for example, to target celiac ganglion 910 and/or other nerve structures.

Transmitter 1602 may have a small size selected to fit into small blood vessels such as the femoral artery for entering the body, and aorta branch vessels. Optionally, transmitter 1602 is located within expandable positioning element 1604 (e.g., balloon). Transmitter 1602 may be aligned at one side of expanded positioning element 1604, or located approximately in the middle of expanded positioning element 1604. Transmitter 1602 may transmit in the opposite direction of the target neural structure, towards a reflector.

Optionally, a reflector 1606 is located within expandable positioning element 1604. Optionally, upon expansion, reflector 1606 portion of positioning element 1604 takes on a preselected size and/or shape to provide focused energy transmission, for example, a concave reflector. Optionally, reflector 1606 is coupled to expandable positioning element 1604 so that expansion of positioning element 1604 expands the reflector, for example, reflector is integrated within the wall of the positioning element, reflector is bonded to the inner wall of the positioning element, or other coupling methods.

Optionally, reflector 1606 is located on the opposite side facing the target neural structure. Transmissions from transmitter 1602 are reflected by reflector 1606 back towards the target neural structure to ablate the neural structure.

Optionally, the diameter of reflector 1606 in the expanded state is larger than the diameter of the vessel in which reflector 1606 is positioned for ablation. Optionally, reflector 1606 is sized and/or positioned to fit within the vessel when expanded, for example, reflector is arranged along the long axis of the vessel.

Optionally, catheter 1600 with collapsed reflector 1606 and collapsed positioning element 1602, and with transmitter 1602 is sized for insertion into small blood vessels, for example, femoral artery, or celiac artery 912, other aorta branch vessels or other lumens. Optionally, expansion of reflector 1606 and positioning element 1602 provide for ablation of the target neural structure by focused energy from within the small diameter blood vessel.

Positioning element 1604 may be filled with fluid to transfer energy from transmitter 1602 to the inner vessel wall, for example, as described with reference to FIG. 14. Transmitter 1602 may be an ultrasound transmitter or other transmitters, for example, as described with reference to FIG. 14. It is expected that during the life of a patent maturing from this application many relevant nerve ablation systems, electrodes, and nerve structure location systems will be developed and the scope of the terms nerve ablation systems, electrodes, and nerve structure location systems are intended to include all such new technologies *a priori*.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A catheter for ablation of a neural structure comprising:
at least one rod sized for insertion into at least one of an aorta and an aorta branch vessel;
a plurality of electrodes located at a distal end portion of the at least one rod, the electrodes disposed in a spaced apart arrangement along the distal end portion, the spacing selected to position at least one of a first of the plurality of electrodes within the aorta or aorta branch vessel when at least one of a second of the plurality of electrodes is positioned within another aorta branch vessel, the spaced apart arrangement selected to encompass at least one neural structure by forming an axis between the at least one first positioned electrode and at least one second positioned electrodes of the plurality of electrodes; and
a controller programmed to apply electrical energy to at least the two of the plurality of electrodes forming the axis, the electrical energy applied in an amount to ablate at least a portion of the at least one neural structure located at least one of along and in proximity to the axis, without significantly ablating tissue surrounding the at least one neural structure.

2. The catheter of any of the previous claims, wherein the at least one neural structure comprises one or more of: celiac ganglion, superior mesenteric ganglion, inferior mesenteric ganglion, aorticorenal ganglion.

3. The catheter of any of the previous claims, wherein the at least one aorta branch vessel comprises at least one of: aorta, celiac trunk, common hepatic artery, left gastric artery, splenic artery, superior mesenteric artery, inferior mesenteric artery.

4. The catheter of any of the previous claims, wherein in proximity to the axis comprises less than about 50 mm from the axis.

5. The catheter of any of the previous claims, further comprising at least one positioning element for one or both of securing the position of the electrodes and urging the plurality of electrodes against an inner wall of aorta and/or aorta branch vessel.

6. The catheter of any of the previous claims, wherein the controller is further programmed to select the at least two of the plurality of electrodes forming an axis from the plurality of electrodes, and applying energy to the selected electrodes.

7. The catheter of any of the previous claims, wherein the curvature of the electrodes is designed to contact the inner wall of the aorta and/or aorta branch vessel.

8. The catheter of any of the previous claims, wherein one or more electrodes are located on each of a plurality of rods, wherein the different rods are designed for positioning inside different aorta branch vessels.

9. The catheter of any of the previous claims, wherein the radial positions of the electrodes along the rod are selected for the electrodes to generally face the at least one neural structure.

10. The catheter of any of the previous claims, wherein the controller is programmed to deliver bipolar RF energy between the electrodes forming the axis.

11. The catheter of any of the previous claims, wherein the at least one neural structure comprises one or more of: celiac ganglion, superior mesenteric ganglion, inferior mesenteric ganglion, aorticorenal ganglion.

12. The catheter of any of the previous claims, wherein the one aorta branch vessel comprises at least one of: aorta, celiac trunk, common hepatic artery, left gastric artery, splenic artery, superior mesenteric artery, inferior mesenteric artery.

13. The catheter of any of the previous claims, further comprising an expandable positioning element containing a reflector and a transmitter, the reflector having an expanded size and shape for reflecting energy transmitted by the transmitter at a focal region containing at least one neural structure at least 5 mm away from a surface of the aorta branch vessel, the reflector coupled to the expandable positioning element so that expansion of the positioning element expands the reflector;
wherein the positioning element has an expanded size and shape for securing the position of the transmitter and reflector inside the aorta branch vessel when the positioning element is in an expanded state, the positioning element located at the distal region of the catheter;
wherein the positioning element is filled with fluid suitable for transmission of focused energy to the inner wall of at least one of the aorta and aorta branch vessel.

14. The catheter of claim 13, wherein the distal end of the catheter is sized for insertion into the aorta branch vessel when the positioning element and the reflector are collapsed, and the diameter of the expanded reflector is larger than the diameter of the aorta branch vessel when expanded.

15. The catheter of claim 13, wherein the transmitter is aligned at one side of the expanded positioning element, or located approximately in the middle of the expanded positioning element, and the transmitter is oriented to transmit in the opposite direction of the target neural structure, towards the reflector.
